# EUROPEAN PATENT APPLICATION

(11) **EP 4 653 543 A1**
(43) Date of publication of application: **26.11.2025**
(21) Application number: 24223666.9
(22) Date of filing: 30.12.2024
(51) Int. Cl.: C12Q 1/6818, C12Q 1/6804

(54) **A LABEL, MARKER AND METHOD FOR ANALYSING A BIOLOGICAL SAMPLE**

(30) Priority: 21.05.2024 EP 24177155
(71) Applicant: Leica Microsystems CMS GmbH, 35578 Wetzlar (DE); ATTO-TEC Gmbh, 57074 Siegen (DE)
(72) Inventor: Alsheimer, Soeren, 35578 Wetzlar (DE); Zilles, Alexander, 57074 Siegen (DE)
(74) Representative: Schaumburg und Partner Patentanwälte mbB

(57) **Abstract**

In a first aspect a label for analysing a biological sample is provided. The label comprises a first label part comprising a first nucleic acid backbone (100, 206), and a second label part comprising a second nucleic acid backbone (102, 210). The first nucleic acid backbone (100, 206) and the second nucleic acid backbone (102, 210) are each configured to hybridise to the respective other one. The label further comprises at least one first labelling moiety (208) and at least one second labelling moiety (212). The label further comprises at least one guest molecule (104) configured to form a complex with a host molecule (106). In further aspects, a corresponding marker (200, 500, 600) and a method for analysing a biological sample are provided.

## Description

### Technical field

The invention relates to a label and a corresponding marker. In a further aspect, a method for analysing a biological sample is provided.

### Background

Labels and markers are frequently used when analysing biological samples, for example in fluorescent microscopy. Markers for fluorescent microscopy generally comprise affinity reagents (AR), such as antibodies, nanobodies, aptamers, affimers, polymeric binders, toxins, and oligonucleotide probes (e.g. FISH probes), and labels attached to the affinity reagents, in order to enable specifically attaching the labels-in particular via the affinity reagents - to a target analyte in a biological sample.

Detection in affinity reagent-based assays is usually performed by marking analytes with markers that generally comprise at least one affinity reagent and a (detectable) label, which may for example be an enzyme, a metal-tag, a Raman label, a fluorescent label or a hybrid label. A fluorescent label may comprise for example a fluorescent dye, a fluorophore, a fluorochrome, a dye, a QDot, a PDot, or a polymer dye. A detectable label may be, in particular, an optically detectable label.

Affinity reagents are used in life science to bind targets or analytes with high affinity and specificity. A variety of assays for liquid or solid samples rely on detection reagents that comprise an affinity reagent and a detectable label, which may comprise a nucleic acid, e.g. an oligonucleotide, a (fluorescent) dye, an enzyme, a metal tag, a radioactive tag, or an affinity tag (e.g. HA-, Myc-, FLAG-tags). Both aptamers, antibodies, and nanobodies have dissociation constants (KD) in the pM to µM range and are used in a variety of affinity reagent-based assays or immunoassays, which are a pillar of life science research.

The various applications of markers include multiplexing approaches, which require large sets of distinguishable markers, whilst detecting analytes that only occur in small quantities generally requires markers with bright labels. In particular when handling large sets of markers, which may be assembled from individual components such as dyes and affinity reagents, it is of interest to reduce complexity of handling, whilst maintaining flexibility of use and reliability.

### Summary

It is an object to provide a label that enables efficient handling when analysing biological samples.

The aforementioned object is achieved by the subject-matter of the independent claims. Advantageous embodiments are defined in the dependent claims and the following description.

In the sense of this document, a sample refers to a biological sample or specimen including for example blood, serum, plasma, tissue, bodily fluids (e.g. lymph, saliva, semen, interstitial fluid, cerebrospinal fluid), faeces, solid biopsy, liquid biopsy, explants, cells (e.g. prokaryotes, eukaryotes, archaea), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document, sample further refers to a volume surrounding a biological sample. For example, in assays, where secreted proteins like growth factors, extracellular matrix constituents are being studied, the extracellular environment surrounding a cell up to a certain assay-dependent distance is also referred to as a sample. Specifically, affinity reagents brought into this surrounding volume are referred to in the sense of this document as being introduced into the sample. Particularly relevant sample types for the present disclosure are Formalin-fixed paraffin embedded tissue sections or tissue microarrays as well as tissue cryosections.

This allows the identification and/or localisation of the target analyte in the biological sample. An affinity reagent in the sense of this document may be for example an antibody, an antibody fragment, a nanobody, an aptamer, an aptabody, a polymeric binder, an affimer, an oligonucleotide probe (e.g. ISH or FISH probe), a drug/drug-like molecule or a toxin. An affinity reagent (AR) may also be a derivative of the above. An affinity reagent is usually configured to bind a target analyte or target with high specificity and affinity. However, many antibodies, which form the largest class of commercially available affinity reagents that bind proteins, are known to exhibit substantial cross-reactivity. In other words, many if not all antibodies do not only bind to their cognate target analyte, but also to a number of OFF-targets, i.e. targets that are not subject to the intended investigation. Both the cognate target against which an affinity reagent was produced or raised and the OFF-target may be collectively referred to as targets.

A further particularly relevant class of affinity reagents are aptamers and their derivatives. Aptamers are typically short nucleic acid sequences that fold into 3-dimensional structures capable of binding a target or analyte with high affinity and specificity. In the sense of this document, an aptamer refers to a small affinity reagent that is typically based on nucleic acids - either naturally occurring RNA, DNA or artificial ones XNAs, but may in some cases also be based on a peptide backbone. In the sense of this document, aptamers also refer to derivatives of aptamers such as SOMAmers, aptabodies, or other aptamer-derivatives, for example modified with modifications that are typically found on proteins such as glycosylation. Aptamers are generated by SELEX, can be produced at low cost with very high batch-to-batch consistency, practically unlimited shelf-life at -20°C, can be easily modified and are well suited to o a cyclic staining and imaging process described the European patent application with the application number 23178065.1, the complete content thereof being incorporated herein by reference. Both aptamers, antibodies, and nanobodies have dissociation constants (KD) in the pM to µM range and are used in a variety of affinity reagent-based assays or immunoassays, which are a pillar of life science research.

Generally, the label may be connected to the primary affinity reagent or a secondary affinity, which binds to the primary affinity reagent and thereby also introduces a certain degree of amplification. An affinity reagent may also be labelled with an oligonucleotide barcode, which can be addressed using a complementary oligonucleotide sequence that may be connected to a label. Using oligonucleotide barcodes and linkers provides an easy and efficient way not only to connect the elements of the marker, but also to incorporate additional functional elements like priming sites that can be used for amplification of barcode sequences by enzymatic amplification (e.g. polymerase chain reaction, PCR; loop-mediated isothermal amplification, LAMP; rolling circle amplification, RCA) or by hybridization chain reaction (HCR). In addition, or alternatively, landing sites for adapters may be incorporated that allow the formation of dendrimeric structures. This allows amplification of the signal, which is especially desirable when low abundance targets like PD1 or PD-L1 shall be analysed in tissue sections that have high background autofluorescence. In the sense of the present disclosure, which provides a novel way for paired detection the label comprises at least a first and second label part, which are connected directly or indirectly to a first and second affinity reagent.

A further challenge with using such markers is the cross-reactivity that affinity reagents display, i.e. they generally do not only bind to their target analyte also referred to as target or cognate target in the following, but may also recognize albeit typically with lower affinity other so called OFF-targets. This is the case for antibodies and other protein-binding affinity reagents as well as for oligonucleotide probes, which are configured to bind to nucleic acid targets.

The label, a marker, and a method described herein allow detecting the proximity of multiple analytes in a biological sample. Said analytes may be part of the same protein in which case the method may be used to study post-translational modifications such as phosphorylation, ubiquitination, and acetylation for example or said analytes may be different proteins in which case the method may be used to determine their proximity in the sample as a proxy for protein-protein interaction. Further, said method may be used to probe multiple epitopes on the same target protein to enhance the specificity of the detection, or may be used to detect the presence of multiple epitopes on the same pathogen e.g. E. coli or virus to determine the respective strain with very high specificity. In other words, the present disclosure provides a proximity assay, which may be referred to as Host-Guest controlled Proximity Hybridization Assay (HGPHA).

Earlier, a solution of this problem was provided that is based on collecting cross-reactivity profiles and computational cross-reactivity unmixing as described in the European patent application with the application number 23190568.8, the complete content thereof being incorporated herein by reference. In the present document, an assay format is proposed that is particularly suitable to detect the binding of pairs of affinity reagents to a target analyte. This method may be used to measure for example a high number of protein-protein-interactions in tissue samples, which is sometimes referred to as "spatial interactomics".

As fields like cytometry, plasma proteomics, and microscopy are evolving towards higher levels of plexity, the problem of cross reactivity has come more into focus and has been recognized by the scientific community as a key component of the so-called reproducibility crisis. In a position paper called "Reproducibility: Standardize antibodies used in research" and published by Bradbury and Pluckthin in Nature 518, 27- 29 (2015) with 110 cosignatories, it was stated that fewer than half of around 6,000 routinely used commercial antibodies recognized only their specified targets.

More recent work by Schwenk et al. (Toward Next Generation Plasma Profiling via Heat-induced Epitope Retrieval and Array-based Assays, Molecular & Cellular Proteomics, Volume 9, Issue 11, 2497 - 2507) analysed 11,000 affinity-purified, monoclonal antibodies and found only 531 to produce a single band on a western blot. While a systematic and comprehensive analysis of the phenomenon has not been performed, the advent of antibody microarrays has generated new possibilities to probe crossreactivity of affinity reagents in a practical assay format. From a conceptual point of view, it is therefore very well possible that we will never be able to make a genuinely specific affinity reagent, i.e. an affinity reagent that truly binds to only one target. Bradbury and Pluckthin (Bradbury, A., Plückthun, A. Reproducibility: Standardize antibodies used in research, Nature 518, 27-29 (2015). https://doi.org/10.1038/518027a) further state that cosignatories were able to replicate the scientific results of only 6 of 53 landmark preclinical studies and went on to predict that this phenomenon is costing an estimated $350 million per year in the United States alone.

### Paired detection

Paired detection, wherein the same analyte or multiple analytes may be detected, may be used in biochemical assays for various means. Typically, antibody pairs are used in conjunction with an assay like for example the proximity extension assay (PEA) from OLINK (Uppsala, Sweden) to detect the presence of an analyte in a liquid like serum with high specificity or proximity ligation assay (PLA) from Navinci (Uppsala, Sweden) to detect proximities of two distinct proteins. Matched antibody pairs for paired detection assays are readily available from suppliers like abcam, which presently supplies more than 1,800 antibody pairs.

The present method disclosed herein may be referred to as **host/guest-controlled Proximity Hybridisation Assay (hgPHA)** is an assay designed to allow faithful detection of analyte proximities in biological samples to either
(A) **increase the specificity** of the assay by mitigating false-positive events arising from cross-reactivity or cross-hybridization
(B) interrogate
   - post-translational modifications,
   - alternative splicing, or
   - proteolytic processing.

Host/guest-controlled Proximity Hybridization Assay (hgPHA) therefore is relevant both in terms of detecting proximities of two distinct molecules (e.g. two different proteins) as well as might be used to detect the same protein using paired-detection for example with two antibodies that recognize two distinct epitopes on the same protein or two sequence stretches on the same RNA or DNA locus.

### Host-guest controlled hybridization as a strategy to inhibit duplex formation during target binding and removing of unbound affinity reagents and label parts

An important aspect of the present disclosure can be regarded in that **host-guest controlled hybridization** can be leveraged **to render the hybridization or duplex formation of the complementary first and second label parts controllable.** This is necessary to avoid label part-mediated aggregation of the affinity reagents e.g. antibodies, in absence of the targets, which would otherwise disturb the assay, and enables forming the duplex only after the unbound marker and label parts have been washed out. In this way, a duplex can only form, when a pair of affinity reagents, e.g. antibodies, is bound to two targets that are in close proximity, wherein close proximity is defined as being in a radius of around up to 1-10, 5-20, 20-100, or 100-1,000nm radius depending on the configuration of the assay. For example, for a co-detection of two surface markers on a bacterium the spatial stringency may be chosen to be lower for example 100-1,000nm. While for an assay for post-translational modifications nanobodies, short linkers, and shorter first and second label parts may be used to bring the spatial stringency, i.e. required analyte proximity to generate a positive test result, into the range of 5-20nm. For the highest spatial stringency, one or both affinity reagent may be a drug, toxin, or other small molecule (e.g. hormone, neurotransmitter) known to bind to the target with high affinity and specificity. In the sense of this document high affinity refers to KD in the range of µM to fM. In the sense of this document an affinity reagent is regarded specific, when the skilled biologist, biochemist, or molecular biologist would regard the affinity reagent as specific. As proteome-wide systematic binding studies generally have not been performed for antibodies or other affinity reagents, it is clear that a specific affinity reagent in the sense of this document may well have OFF-targets that it binds to although typically less strong than to its cognate target.

### Detection of the duplex formation and thereby label formation

The label of the present disclosure, which may also be referred to as an hgPHA-label, comprises a first label part and a second label part. These label parts further comprise a first nucleic acid backbone and a second nucleic acid backbone, for example oligonucleotide backbones configured to hybridize with each other. To inhibit the formation of the duplex between the first and second label part either one of them or both of them are modified and comprise at least one guest molecule configured to complexate with a host molecule, wherein both the guest and host molecule might be configured such that the complexation leads to strand invasion and a lowering of the melting temperature of the duplex. hgPHA assays according to the present disclosure might in particular be configured such that
- the number of guest molecules per nt of nucleic acid backbone,
- the melting temperature of the duplex under the complexing (Tmc) and decomplexing (Tmdc) condition as well as,
- the ionic strengths of the complexing and decomplexing buffers as well as,
- the concentration of potential additives such as formamide, dextran, competitor guest molecules, cap molecules, and
- the temperature under which the binding of label parts to the analytes in the biological sample as well as,
- the temperature under which the duplex formation or hybridization of the first and second label parts is achieved,
are selected such or tuned that they fit to the sample type (e.g. lysate vs. cellular samples, bacteria, cell culture; vs. tissue sections) and to the intended application optimally. In other words, some samples tolerate high temperatures in the range of 65-95°C well (e.g. diagnostic testing for bacterial or viral pathogens), whereas others like most tissue sections (e.g. FFPE tissue sections) should not be heated to more than 40-60°C due to build up of autofluorescence. This is further detailed in the following.

hgPHA has a number of advantages over the prior art: (1) The change from complexing condition (inhibition of duplex formation) to decomplexing (allowing duplex formation) is rapid and easy for example by simple washout and preferably competition. (2) Both detection mechanism FRET and dequenching (of a labelling moiety, e.g. a fluorescent label) are robust and can be easily readout on fluorescence readers, fluorescence gel documentation systems, spectrometers, fluorometers, cytometers, FACS-devices, microscopes. (3) The first and second label part can be configured such that they allow single molecule detection. (4) Only little hands-on-time is required. (5) Expensive enzymes are not required. (6) Nevertheless, hgPHA is compatible with various enzymatic and non-enzymatic amplification approaches. (7) In addition to fluorescence intensity change, for FRET-based detection hgPHA can also be used on devices like the STELLARIS confocal microscope from Leica Microsystems (Mannheim, Germany), which is equipped with white-light laser (WLL) pulsed light source and FALCON fluorescence lifetime imaging module to measure the change in fluorescence lifetime (FLIM-FRET; further detail below).

### Terms and Definitions

In the sense of this document, the terms **"fluorescent dye", "fluorophore", "fluorochrome", "dye"** are used interchangeably to denote a fluorescent chemical compound or structure and can be in particular one of the following: a fluorescent organic dye, a fluorescent quantum dot, a fluorescent dyad, a fluorescent carbon dot, graphene quantum dot or other carbon-based fluorescent nanostructure, a fluorescent protein, a fluorescent DNA origami-based nanostructure. From the organic fluorescent dyes in particular derivatives of the following are meant by the term "fluorescent dye": xanthene (e.g. fluorescein, rhodamine, Oregon green, Texas), cyanine (e.g. cyanine, indocarbocyanine, oxacarbocyanine, thiacarbocyanine, merocyanine), derivatives, squaraine rotaxane derivatives, naphthalene, coumarin, oxadiazole, anthracene (anthraquinones, DRAQ5, DRAQ7, CyTRAK Orange), pyrene (cascade blue), oxazine (Nile red, Nile blue, cresyl violet, oxazine 170), acridine (proflavine, acridine orange, acridine yellow), arylmethine (auramine, crystal violet, malachite green), tetrapyrrole (porphin, phthalocyanine, bilirubin), dipyrromethene (BODIPY, aza-BODIPY), a phosphorescent dye, or a luminescent dye. The following trademark groups denote commercially available fluorescent dyes, which may include dyes belonging to different chemical families such as CF dye (Biotium), DRAQ and CyTRAK probes (BioStatus), BODIPY (Invitrogen), EverFluor (Setareh Biotech), Alexa Fluor (Invitrogen), Bella Fluore (Setareh Biotech), DyLight Fluor (Thermo Scientific), Atto and Tracy (Sigma-Aldrich), FluoProbes (Interchim), Abberior Dyes (Abberior Dyes), Dy and MegaStokes Dyes (Dyomics), Sulfo Cy dyes (Cyandye), HiLyte Fluor (AnaSpec), Seta, SeTau and Square Dyes (SETA BioMedicals), Quasar and Cal Fluor dyes (Biosearch Technologies), SureLight Dyes (Columbia Biosciences), Vio Dyes (Milteny Biotec). From the group of fluorescent proteins in particular the members of the green fluorescent protein (GFP) family including GFP and GFP-like proteins (e.g DsRed, TagRFP) and their (monomerized) derivatives (e.g., EBFP, ECFP, EYFP, Cerulaen, mTurquoise2, YFP, EYFP, mCitrine, Venus, YPet, Superfolder GFP, mCherry, - 5 - mPlum) are meant by the term "fluorescent dye" in the sense of this document. Further from the group of fluorescent proteins the term "fluorescent dye" in the sense of this document may include fluorescent proteins, whose absorbance or emission characteristics change upon binding of ligand such as BFPms1 or in response to changes in the environment such as redox-sensitive roGFP or pH sensitive variants. Further from the group of fluorescent proteins the term "fluorescent dye" in the sense of this document may include derivatives of cyanobacterial phycobiliprotein small ultra red fluorescent protein smURFP as well as fluorescent protein nanoparticles that can be derived from smURFP. An overview of fluorescent proteins can be found in the article of Rodriguez et al. in Trends Biochem Sci. 2017 Feb; 42(2): 111-129. A fluorescent dye in the sense of this document may further refer to a fluorescent quantum dot. A fluorescent dye in the sense of this document may further refer to fluorescent carbon dot, a fluorescent graphene quantum dot, a fluorescent carbon-based nanostructure as described in the article of Yan et al. in Microchimica Acta (2019) 186: 583 and Iravani and Varma 2020 in Environ Chem Lett. 2020 Mar 10 : 1-25. A fluorescent dye in the sense of this document may further refer to a fluorescent polymer dot (Pdot) or nanodiamond. A fluorescent dye in the sense of this document may further refer to a fluorescent dyad, such as a dyad of a perylene antenna and a triangelium emitter as described in the article of Kacenauskaite et al. in J. Am. Chem. Soc. 2021, 143, 1377-1385. A fluorescent dye in the sense of this document may further refer to an organic dye, a dyad, a quantum dot, a polymer dot, a graphene dot, a carbon-based nanostructure, a DNA origami-based nanostructure, a nanoruler, a polymer bead with incorporated dyes, a fluorescent protein, an inorganic fluorescent dye, a SMILE, or a microcapsule filled with any of the aforementioned. A fluorescent dye in the sense of this document may further refer to a FRET-pair having at least one fluorescent dye as FRET donor and at least one fluorescent dye as a FRET acceptor, or a FRET-triple, which is used to generate a three component Förster resonance energy transfer. In particular, the FRET-pair or FRET-triplet is connected by a complementary linker or by a linking element. A fluorescent dye in the sense of this document may further refer to a FRET n-tupel of physically connected dyes. A fluorescent dye may also be a polymer dye.

**Förster Resonance Energy Transfer (FRET)** has been widely used to measure protein-protein interactions using both fluorescent proteins and fluorescent dyes. The spatial stringency of FRET is in the range of 10nm. In other words, the FRET efficiency E E= 1/(1+(r/R0)^6 ) drops exponentially with an increase in donor and acceptor dye distance r, such that effective FRET typically occurs when the donor and acceptor dye are within 1 to 10nm distance r to each other, wherein R0 is the Förster Distance of the donor-acceptor dye pair. Typically, the donor and acceptor dye or fluorophore are different fluorophores. For example the following dye pairs are commonly used FRET pairs: ATTO 425 - ATTO 520, ATTO 488 - ATTO 550, ATTO 488 - ATTO 565, ATTO 488 - ATTO 647N, ATTO 488 - ATTO 655, ATTO 520 - ATTO 647N, ATTO 532 - ATTO 647N, ATTO 532 - ATTO 655, ATTO 550 - ATTO 590, ATTO 550 - ATTO 647N, ATTO 565 - ATTO 590, ATTO 565 - ATTO 647N, ATTO 590 - ATTO 620, ATTO 590 - ATTO 647N, ATTO 590 - ATTO 680, ATTO 620 - ATTO 680, Alexa Fluor 488 - Alexa Fluor 555, Alexa Fluor 594 - Alexa Fluor 647, Fluorescein - Tetramethylrhodamine. Likewise, commonly used fluorescent protein FRET pairs exists including but not limited to: ECFP-EYFP, mTurquoise2-mVenus, EGFP-mCherry, mNeonGreen-mRuby3. Quantum dots may also be used as donor or acceptors. The use of quantum dots as FRET donors is particularly advantageous because of the high extinction coefficients, which leads to brighter FRET signals. Various methods exist to measure the FRET efficiency including but not limited to measuring the intensity of the acceptor emission (sensitized emission), photobleaching FRET wherein the donor is bleached and bleaching rates differ depending on the presence or absence of the acceptor, fluorescence lifetime measurements that register the change in the lifetime of the donor (i.e. when FRET occurs the donor lifetime shortens; FRET-FLIM). Likewise, FRET may be assessed by measuring donor dequenching following the removal or bleaching of the acceptor (e.g. acceptor photobleaching). In addition to these methods FRET may also be measured by anisotropy imaging like for example in the case of Homo-FRET, i.e. in assays wherein the donor and acceptor are of the same fluorophore. While these aforementioned methods generally readout pluralities of donor and acceptor dye molecules, methods to measure single molecule FRET have likewise been developed. A simple method to measure FRET is ratiometric imaging of donor and acceptor intensity (sensitized emission), which can be performed on simple channel-based readouts like widefield microscopes, cytometers or plate readers. While measuring FRET efficiency or FRET signal in this way by measuring the donor and acceptor emission is simple, it is connected to the challenge of donor crosstalk into the acceptor emission channel, which may also be named the FRET channel. This is why more sophisticated methods like measurement of the donor dequenching were developed. Alternatively, spectral imaging offers a good solution to the crosstalk problem in FRET measurements, but suitable instrumentation may not always be available. In tissue sections, however, it is difficult to perform FRET measurements, because of the high background autofluorescence. While FRET has been performed in tissue sections before, it is known in the field and also evident from the published data that FRET-based assays in tissue sections are limited by poor signal-to-noise or signal-to-background, because of the high background autofluorescence that is commonly found in tissue samples. Reference is made to König P, Krasteva G, Tag C, König IR, Arens C, Kummer W., FRET-CLSM and double-labelling indirect immunofluorescence to detect close association of proteins in tissue sections, Lab Invest. 2006 Aug;86(8):853-64. doi: 10.1038/labinvest.3700443. Epub 2006 Jun 19. PMID: 16783395.

The present disclosure, therefore, proposes an assay format that combines bright labelling for easy detection with high specificity in a way that is compatible with a cyclic staining process for high plex analysis of with improved specificity, for high plex analysis of molecular interactions and/or posttranslational modifications. The present disclosure is suited for any kind of analyte and AR.

In a first aspect a label for analysing a biological sample is provided. The label comprises a first label part comprising a first nucleic acid backbone, and a second label part comprising a second nucleic acid backbone. The first nucleic acid backbone and the second nucleic acid backbone are each configured to hybridise to the respective other one. In particular, the first nucleic acid backbone and the second nucleic acid backbone may be at least partially complementary to each other. Hybridisation of the first nucleic acid backbone and the second nucleic acid backbone may result in the formation of a duplex or double stranded nucleic acid. The label further comprises at least one first labelling moiety and at least one second labelling moiety. The label further comprises at least one guest molecule configured to form a complex with a host molecule.

The label may be used for detection of the proximity between two target molecules, for example.

In particular, the label may be configured to be optically detectable at least when the first label part and the second label part are in close proximity. For example, the at least one first labelling moiety and/or the at least one second labelling moiety may be optically detectable, such as fluorophores, and the optical properties of at least one of the first labelling moiety and the second labelling moiety may change depending on the distance between the first labelling moiety and the second labelling moiety, or the distance between the first label part and the second label part. In particular, the at least one first labelling moiety and the at least one second labelling moiety may have the same optical properties, such as excitation wavelength, emission wavelength and fluorescence lifetime, or they may have different optical properties. The first labelling moiety and the second labelling moiety may be attached to the nucleic acid backbone of the same label part or to the nucleic acid backbones of different label parts of the label.

The guest molecule is preferably covalently attached to one of the nucleic acid backbones. In particular, the label may comprise a plurality of guest molecules that are attached to either or both nucleic acid backbones. In this case, one guest molecule may bind to one host molecule. In particular, the at least one guest molecule is not a nucleotide and/or not a labelling moiety. In a particular embodiment, there may be one guest molecule for every ten nucleotides of one of the nucleic acid backbones.

The guest molecule is preferably configured to selectively form a complex with the host molecule. Thus, the guest molecule may be configured to form the complex with the host molecule only under a particular complexing condition. Similarly, the guest molecule may be configured to decomplex from the host molecule only under a decomplexing condition, which is different to the complexing condition. In the simplest case the complexing condition is characterised by presence or a high concentration (e.g. µM-mM range) of host molecule and the decomplexing condition is characterized by a low concentration or absence of the host molecule. The complex formation may include binding of the guest molecule to the host molecule. For example, the complex formation may be based on intermolecular forces such as hydrogen bonding, Van der Waals forces, or dipole interactions. Preferably, the complex formed between the host molecule and the guest molecule is configured to disrupt or hinder hybridisation of the first nucleic acid backbone and the second nucleic acid backbone to each other. In particular, the complex may reduce the melting temperature of the duplex of the hybridised first and second nucleic acid backbone.

Preferably, the at least one guest molecule is configured to form the complex with the host molecule under a complexing condition. Similarly, the guest molecule and/or the host molecule are configured to decomplex under a decomplexing condition. Such a decomplexing condition and/or complexing condition may include varying a concentration of the host molecule or addition of a competitor guest molecule, varying pH, salt, and/or temperature. This enables selectively forming the complex between the host molecule and the guest molecule. In particular, the complexation of the at least one guest molecule with the host molecule may lead to a strand invasion and a dissociation of the duplex between the first and second nucleic acid backbone.

Preferably, the optical properties of at least one of the first labelling moiety and the second labelling moiety change depending on the distance between the first label part and the second label part, in particular depending on the distance between the first labelling moiety and the second labelling moiety. In particular, the change in optical properties may be detected in order to determine the distance between the label parts. The change in the optical properties may be proportional to the distance between the label parts.

Preferably, the complex is configured to invade the duplex and to reduce its melting temperature.

Preferably, the first nucleic acid backbone and the second nucleic acid backbone each comprise at least one of a natural nucleic acid and a nucleic acid analogue. In particular, the first nucleic acid backbone and/or the second nucleic acid backbone may consist essentially of a nucleic acid analogue. The first nucleic acid backbone and/or the second nucleic acid backbone may comprise a single nucleic acid molecule.

The first nucleic acid backbone and the second nucleic acid backbone are at least partially complementary to each other, in particular, to hybridise and form a duplex. The guest-host complex formation may hinder this hybridisation. The guest molecule is preferably (covalently) attached to a phosphate backbone or, or a ribose, or a nucleobase of the respective nucleic acid backbone. In some embodiments the first nucleic acid backbone or the second nucleic acid backbone may comprise an XNA or xeno nucleic acid like a peptide nucleic acid, in this case the guest molecule may be conjugated to the peptide backbone of said PNA.

Preferably, one of the first nucleic acid backbone and the second nucleic acid backbone consists essentially of a nucleic acid sensitive to a degradation agent, for example, a natural nucleic acid, and the other one of the first nucleic acid backbone and the second nucleic acid backbone consists essentially of a nucleic acid analogue resistant to the degradation agent. This enables selective degrading one or all of the first and second nucleic acid backbones, for example, in an iterative staining process.

Such a degradation agent may be DNase I or a restriction enzyme. In some embodiments that nucleic acid backbones may comprise a cleavage site configured to be cleaved either chemically (e.g. reductive cleavage with TCEP, hydrogen peroxide) or physically (e.g. via UV light, temperature). It is clear that these examples are provided here merely for the sake of providing examples. The skilled person will easily find more chemistries and/or enzymes that can be used to render the label or parts thereof degradable.

Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases.

Further, in case an affinity reagent comprises or consists essentially of nucleic acids, these nucleic acids may similarly be nucleic acid analogues resistant to the degradation agent.

Preferably, the at least one first labelling moiety and/or the at least one second labelling moiety is optically detectable. This enables efficient detection of the label, for example, by means of a microscope, in particular a fluorescence microscope. For example, the first labelling moiety and/or the second labelling moiety may comprise a fluorophore such as fluorescent proteins, organic or inorganic fluorescent molecules, or fluorescent nanoparticles.

Preferably, the at least one first labelling moiety and/or the at least one second labelling moiety preferably comprise identical fluorescent dyes. In particular, the fluorescent characteristics of the at least one first labelling moiety and/or the at least one second labelling moiety are essentially the same. Alternatively, the at least one first labelling moiety comprises at least one first fluorescent dye and the at least one second labelling moiety comprises at least one second fluorescent dye, the at least one first fluorescent dye and the at least one second fluorescent dye having different characteristics. The characteristic may be different regarding the excitation, emission and/or fluorescent lifetime of the at least first and second fluorescent dye.

Preferably, the at least one first labelling moiety and the at least one second labelling moiety are configured for non-radiative energy transfer between them, in particular when they are in close proximity to each other. For example, the first labelling moiety and the second labelling moiety may form a fluorescence resonance energy transfer (FRET) pair, where one is a FRET donor and the other is a FRET acceptor. This enables determining whether or not the first label part and the second label part are in close proximity to each other based on the FRET efficiency between the FRET pair. Generally, the FRET efficiency is highly dependent on the distance between the FRET donor and the FRET acceptor. A close proximity in this context may be characterised based on this to be in a range between 1 and 10 nm. In a particular example, the FRET donor and the FRET acceptor may have different optical properties, such as emission or excitation wavelengths, when no FRET occurs. This enables accurately determining whether or not an energy transfer occurs between the FRET acceptor and donor pair. Preferably, the FRET donors as the first labelling moieties might be arranged on the first label part or on the first nucleic acid backbone and the FRET acceptors as the second labelling moieties might be arranged on the second label part or on the second nucleic acid backbone. Thus, FRET pairs might be formed in case the first nucleic acid backbone and the second nucleic acid backbone hybridise at least partially together, thereby forming a duplex, as described further below.

In an alternative example, the first labelling moiety and the second labelling moiety may be of the same type, meaning that they have the same optical properties. In this case, self-quenching between the first labelling moiety and the second labelling moiety may occur, in particular when the proximity of the first labelling moiety and the second labelling moiety leads to non-radiative energy transfer among them. This may significantly diminish the emission efficiency and the fluorescence intensity that can be detected from the first labelling moiety and the second labelling moiety. Thus, in this case, the proximity of the first label part and the second label part may be detected by a reduced fluorescence intensity when the first label part and the second label part are in close proximity. In this example, the first labelling moiety and the second labelling moiety might be arranged on the same nucleic acid backbone, i.e. on the first nucleic acid backbone or on the second nucleic acid backbone, as described further below.

With reference to the above, where the first labelling moiety and the second labelling moiety are configured for non-radiative energy transfer between them, the first labelling moiety and the second labelling moiety may be attached to the respective first or second nucleic acid backbone. Thus, the first labelling moiety may be attached to the first nucleic acid backbone and the second labelling moiety may be attached to the second nucleic acid backbone.

Alternatively, the first labelling moiety and the second labelling moiety may be attached to the same nucleic acid backbone. Thus, the first labelling moiety and the second labelling moiety may only be attached to the first nucleic acid backbone or the second nucleic acid backbone. In this case, the first and second labelling moieties are preferably hydrophobic labelling moieties.

Generally, in an aqueous solution hydrophobic labelling moieties have a tendency to aggregate. In particular, hydrophobic labelling moieties that are arranged in close proximity on a flexible single stranded nucleic acid backbone may lead to formation of aggregates. This aggregation may lead to self-quenching or changes to the optical properties of these labelling moieties such as their absorption wavelength and/or in particular their fluorescence emission or fluorescence brightness, compared to non-aggregated hydrophobic labelling moieties. The formation of the duplex between the first and second nucleic acid backbones when they are in close proximity results in an increase in the degree of rigidity or stiffness of the nucleic acid backbone. A measure of the degree of rigidity or stiffness of a nucleic acid backbone, in particular of a duplex structure, is persistence length (L_{P}). The persistence length is a mechanical parameter quantifying polymer rigidity: the higher the persistence length, the more rigid the polymer. In the presence of monovalent or divalent salts, a nucleic acid duplex structure regularly has a persistence length of 30 to 55 nm, while single stranded nucleic acid is much more flexible, with a persistence length of 1.5 to 3 nm. Thus, a double stranded nucleic acid has a higher persistence length and correspondingly a more linear form.

In particular, when the first labelling moiety and the second labelling moiety are attached on opposing ends of the first or second nucleic acid backbone the increase in rigidity of the respective nucleic acid backbone may result in an increase in the distance between the first and second labelling moiety. Thus, previously aggregated labelling moieties are separated by hybridisation of the first and second nucleic acid backbones. This separation may result in a detectable change in their optical properties.

Examples of hydrophobic labelling moieties that are regularly used to analyse biological samples include ATTO 390, ATTO 425, ATTO 550, ATTO Rho12, ATTO 633, and ATTO 647N.

Preferably, the at least one first labelling moiety and the at least one second labelling moiety are attached to either the first nucleic backbone or the second nucleic backbone. For example, the labelling moiety may be attached, preferably covalently, to the phosphate backbone of the respective nucleic acid backbone. In particular, the first labelling moiety and the second labelling moiety may be attached to opposite ends of one of the first and second nucleic acid backbones.

In an alternative embodiment, the at least one first labelling moiety is attached to the first nucleic acid backbone and the at least one second labelling moiety is attached to the second nucleic acid backbone.

Preferably, the first nucleic acid backbone extends along a first direction and a plurality of the first labelling moieties are arranged on the first nucleic acid backbone along the first direction, and/or the second nucleic acid backbone extends along a second direction and a plurality of the second labelling moieties are arranged on the second nucleic acid backbone along the second direction. This enables generating an optically detectable signal that is proportional to the distance between the first label part and the second label part. In particular, the respective labelling moieties are arranged one after another along the respective direction.

Preferably, each labelling moiety is substantially equally spaced from any adjacent labelling moiety. This enables generating an optically detectable signal that is proportional to the distance between the first label part and the second label part. When the label comprises plurality of labelling moieties each first labelling moiety is substantially equally spaced from any adjacent first labelling moiety, and/or each second labelling moiety is substantially equally spaced from any adjacent second labelling moiety. Preferably, the essentially equal spacing may be in a range from 0.33nm to 33 nm.

Preferably, the label comprises a plurality of the guest molecules, wherein the guest molecules are evenly spaced along the first nucleic acid backbone and/or the second nucleic acid backbone. This enables efficient control over the hybridisation of the first nucleic acid backbone and the second nucleic acid backbone by means of the host molecule. In particular, the guest molecules are arranged along the first or second direction. Preferably, one guest molecule is provided per every 5 to 10 nucleotides of the respective nucleic acid backbone. Preferably, the nucleic acid backbone is in the range of 20-120nt in length, in particular, as up to about 120nt can be synthesized with good yield and high quality. In some cases, the nucleic acid backbone may be longer and comprise for example 120nt-600nt, which may be achieved by concatenation or chemical ligation. Reference is made to the European patent application with the application number 24151783.8, the complete content thereof being incorporated herein by reference.

Preferably, the at least one guest molecule is one of 1-adamantanemethylamine, ferrocenyl methylamine, 1,4-benzenedimethanamine, and 4-tertbutylbenzylamine. Xiao et al, 2022 (Controllable DNA hybridization by host-guest complexation-mediated ligand invasion. Nature Communications 13: 5936) provides further details of suitable host- and guest-molecules.

Preferably, the label comprises at least one host molecule. Providing the label with the at least one guest molecule and host molecule enables avoiding random or spontaneous hybridisation of the first nucleic acid backbone and the second nucleic acid backbone. In particular, this enables controlling the hybridisation between the first and second nucleic acid backbone. This makes the introduction of the label parts into a biological sample easier and more efficient. In particular, the label may comprise a plurality of host molecules to at least form a complex with each guest molecule. The host molecule may be configured to form a complex with the guest molecule.

Preferably, the host molecule is a cucurbit[n]uril, in particular cucurbit[7]uril. In particular, the host molecule does not comprise nucleotides or nucleic acids.

With reference to cucurbit[7]uril, which is a particularly preferred host molecule for the method disclosed in this document, the following publication is cited: *A reference scale of cucurbit[7]uril binding affinities.* Alnajjar et al. 2021 Org. Biomol. Chem., 2021, 19, 8521-8529. As detailed in Alnajjar et al. who have used competitive titrations a large range of suitable molecules may be guests for CB[7] spanning the millimolar (e.g. TBA) to femtomolar (e.g. AMADA) range (compare Scheme 1 and Table 1). Several fluorescent dye molecules, which may be (primarily) labelling moieties in the sense of this document, may be guests to CB[7], although not with very high affinity binding. In the sense of the present document, nM binding constants might be regarded as high affinity, pM as very high and fM and higher as ultra-high affinities; unless specified otherwise when "high affinity" is referenced this includes very high and ultra-high affinities.

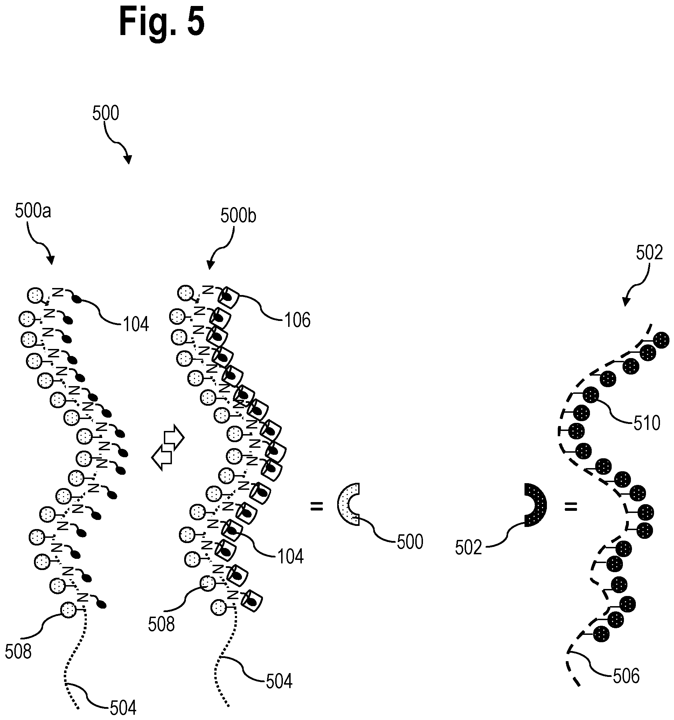

With regard to selecting a guest molecule it is important to note that the method may be carried out in a way, in which the guest molecule has an affinity in the µM to nM range. In this case the complexing buffer contains a µM or mM concentration of CB[7] for example. Similar to the conditions that were used in Figure 5b (10µM CB[7], 30µM FC guest competitor, 15 nt duplex with 3 FC-guest modified cytosines) from Xiao et al. 2022 (Controllable DNA hybridization by host-guest complexation-mediated ligand invasion, Nature Communications 13, 5936), which demonstrated cyclical duplex formation.

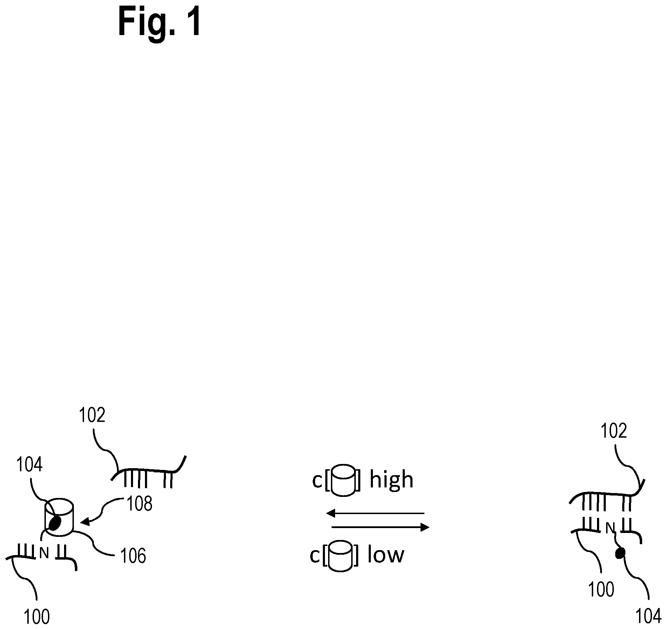

In other words, during the complexing condition the concentration of the host molecule is high such that the majority of guest molecules is complexated with the host molecules and abolishes the duplex formation, which may otherwise mediate the binding of the first and second nucleic acid backbone in solution leading to false-positives. Following to the binding of the marker the decomplexing condition is applied. In the simplest case, this may be a simple wash-out of the host molecule with standard wash buffers like PBS, PDT, the buffer used for blocking which may comprise BSA, dextran, salmon sperm, scrambled DNA for example. The wash-out or buffer exchange effectively lowers the concentration of the host molecule. Alternatively, or in addition a decomplexing buffer may be used to bring about the decomplexing condition, which contains a competing guest molecule, which may be the same or a different guest molecule. For example, if FC (ferrocene or ferrocene-derivatives) is used as a guest molecule, then AD (adamantane or adamantane-derivatives), which have a higher affinity to CB[7], may be used in the decomplexing buffer. Further to this point it is known that *"1-adamantanemethylamine (AD), ferrocenyl methylamine (FC), 1,4-benzenedimethanamine (BA) and 4-tertbutylbenzylamine (TB)"* span *"a wide range of binding affinities on CB[7] (Ka values of previously reported cation derivatives of these guest molecules were ~10¹⁴M⁻¹ for AD, ~10¹²M⁻¹ for FC, ~10⁹M⁻¹ for BA and ~10⁶M⁻¹ for TB)",* but also that *"as shown by the isothermal titration calorimetry (ITC) analysis (Supplementary* *Fig. 1**), the binding constants between CB[7] and these four guest molecules were 1.64* × *10⁹M⁻¹ for AD, 4.70 × 10⁸M⁻¹ for FC, 8.88* × *10⁵M⁻¹ for BA and 3.98 × 104 M-1 for TB, which were significantly reduced mostly due to the effects of pH and the interactions between sodium cations and the carbonyl portals of CB[7]"* cited from Xiao et al. 2022. It is therefore clear to the skilled person that the guest and host molecule combinations as well the concentrations of them as well as of guest molecule competitors as well as concentrations of salts and other buffer constituents provided in this document is provided merely for the sake of example and that many other configurations may be chosen to implement the method without deviating from this invention.

The difference in melting temperatures (Tm) between the complexing condition under which the first and second label parts are attached to their respective targets and the decomplexing condition may be tuned to the desired point by (A) the selection of the guest molecule, (B) the number of guest molecules conjugated to the first and/or second label part, as well as by (C) changing salt concentrations or the addition of competitor guest molecules or other additives like formamide, dextran sulfate or other agents to either complexing, decomplexing, wash, or blocking buffers.

With respect to cucurbiturils as host molecules: In addition to the CB guest molecules, which in the context of this application are to be understood primarily as endo guests, i.e. molecules that are either completely or partially accommodate inside of the host molecule. There are also molecules, which can cap CB molecules that may serve as further additives as they can modify the binding and release kinetics of endo guest molecules. An example for such a molecule is hexaiodobenzene, which was found by computational analysis to bind CB7's portal as described in Lambert et al. npj Computational Materials (2022) 8:21 Supramolecular gating of guest release from cucurbit[7]uril using de novo design.

The following compositions are provided for the sake of providing an example. The person skilled in the art will easily find new additives and make changes to salt concentrations, blocking agent concentrations, chaotropic agents and the like to adapt these compositions to the needs of the particular application or biological sample without deviating from the invention.

### Example Blocking buffer:

### Base component:

5 % normal goat serum/PBS or
5 % normal donkey serum/PBS or
1 % BSA/PBS
137mM - 500mM NaCl
0.2-10mg/ml Salmon sperm DNA (e.g. from THERMO #AM9680)
1-10nmol/ml Scrambled oligonucleotide ssDNA (e.g. from IDT, Iowa)

### Optional further additives:

DMSO
Detergents (Triton-X100, NP-40, saponin, Tween-20)

### Example Decomplexing buffer (allowing duplex formation):

In the simplest case the decomplexing buffer may be just PBS or **Blocking buffer** to washout the host molecule.

Alternatively, it the decomplexing buffer may further comprise a guest molecule competitor. This may be the same as the one used for modifying the nucleic acid backbone of the label part(s) or a different one. For example, if ferrocene (FC) was used to modify the label, FC or a different guest molecule e.g. adamantane (AD) may be included in the decomplexing buffer.
1 % BSA/PBS
137mM - 500mM NaCl
0.2-10mg/ml Salmon sperm DNA (e.g. from THERMO #AM9680)
1-10nmol/ml Scrambled oligonucleotide ssDNA (e.g. from IDT, Iowa)
10µM-1mM Adamantane

### Example Complexing buffer (inhibiting duplex formation):

1 % BSA/PBS
137mM - 500mM NaCl
0.2-10mg/ml Salmon sperm DNA (e.g. from THERMO #AM9680)
1-10nmol/ml Scrambled oligonucleotide ssDNA (e.g. from IDT, Iowa)
10µM-10mM CB[7]

### Example 1: Host-Guest controlled Proximity Hybridization Assay (HGPHA) for direct detection of analyte proximity in cells:

### Preparation:

(A) Prepare oligonucleotide barcoded primary antibody pairs. These may be either procured directly from various vendors or can be generated using available antibody oligonucleotide conjugation kits (e.g. Oligonucleotide Conjugation Kit **ab218260** from abcam (Cambridge, UK)). Oligonucleotides with suitable functional groups may be procured from IDT (Coralville, USA). Likewise, oligonucleotide barcoded primary antibodies may be obtained by using site-specific conjugation.
(B) Prepare first and second label parts comprising complementary barcodes to allow later conjugation to antibody pairs mentioned in (A) via hybridization. This is a particularly easy and efficient way of working with the method. Nevertheless, it is also possible to conjugate the first and second label parts directly and covalently using the aforementioned kit or other chemistries like click chemistry and suitably bi-functional linkers (e.g. NHS-PEG(n)-N3 linkers).
(C) Depending on the expression level(s) of the target(s) it may be desirable to amplify the signal. This may be easily achieved in at least two different ways: (1) by using dendrimeric oligonucleotide-based or other DNA-scaffold structures e.g. nanorulers, DNA origami-, DNA-brick-based structures, (2) by amplifying the barcode sequences using an enzymatic method like rolling circle amplification (RCA), or loop-mediated isothermal amplification (LAMP), or polymerase chain reaction (PCR) for example.

### Main protocol:

1. Wash the cells twice.
2. Fix with 4 % formaldehyde for 10 minutes and wash 3 ×.
3. Permeabilize with 0.1 % TX-100/PBS for 15-20 minutes and wash 3 ×.
4. Block with for 45 minutes in blocking buffer.
5. Dilute the oligonucleotide barcode-conjugated primary antibodies in **Blocking buffer** and apply it for 1-2 h (or overnight at 4 °C). Typically, dilutions are in the range of 1:100-1:500.
6. Wash 4 × thoroughly to remove unbound primary antibody with PBS or **Blocking buffer** each with agitation.
7. Dilute first and second label parts comprising complementary barcode sequences (i.e. complementary to the barcodes on the primary antibodies) in **Complexing buffer.**
8. Apply first and second label parts diluted in **Complexing buffer** to the sample and allow to hybridize to the respective barcodes on the target-bound primary antibodies. This step may be carried out at room temperature for 5-60min or at a higher temperature.
9. Wash 4x with **Complexing buffer** to remove unbound first and second label parts each with agitation. During this step one may also include DAPI for nuclear staining.
10.Optionally, perform a first readout and/or image the sample before applying the decomplexing condition. This readout/image may be used as a baseline and may improve the quality of the assay, but is not required in many cases.
11. Equilibrate the sample to the decomplexing condition by washing the sample 4x or flushing the sample with a suitable volume (e.g. 4-5 flow cell volumes) of **Decomplexing buffer.**
12.Allow the hybridization of the first and second label parts to occur under the decomplexing condition, which may typically require 5-60min.
13.Optionally, provide a drop of mounting medium on a microscope slide and lay the coverslip with the cells or the biological sample upside down on this drop. Press the specimen with the tweezers slightly so that the mounting medium is well distributed, without squeezing the sample. The preparation is ready for microscopy after curing.
14. For optical-grade carrier bottom plates, dishes, or flow cells samples may be imaged directly in the **Decomplexing buffer,** in PBS, or any other imaging buffer of choice, so as long as the imaging buffer does not contain anything that disturbs the formed duplex between the first and second label parts.
15. Perform a readout and/or image the sample.
16.Optionally, perform a comparison of the pixel intensities before and after allowing the duplex formation between the first and second label parts, i.e. under the complexing condition and decomplexing condition to calculate a fold change or ratio of the (FRET) signal on a pixel-per-pixel basis to assess the degree of proximity of the markers and thereby of the analytes.

### Example 2: Host-Guest controlled Proximity Hybridization Assay (HGPHA) for indirect detection of analyte proximity in cells:

1. Wash the cells twice and use tweezers to carefully place the coverslip with upturned cells into the humidified chamber.
2. Fix with 4 % formaldehyde for 10 minutes and wash 3 ×.
3. Permeabilize with 0.1 % TX-100/PBS for 15-20 minutes and wash 3 ×.
4. Block with 5% normal goat serum/PBS or 1 % BSA/PBS for 45 minutes (no washing required).
5. Dilute the primary antibodies in blocking buffer and apply it for 2 h (or overnight at 4 °C). Typically, dilutions are in the range of 1:100-1:500.
6. Wash 4 × thoroughly to remove unbound primary antibody each with agitation.
7. Wash 1x with complexing buffer to equilibrate the sample to the complexing condition with agitation.
   1. Option A: Incubate with the corresponding pair(s) of secondary antibodies diluted in **Complexing Buffer,** which comprise the corresponding first and second label parts for 1-3h, diluted in complexing buffer. Then aspirate the secondary antibody and, if desired incubate with Hoechst or DAPI before proceeding to the washing step 8. Note: Performing the binding of the antibodies comprising label parts is performed under the complexing condition to avoid the hybridization of label parts in solution, which would generate false-positive results.
   2. Option B: Incubate with the corresponding pair(s) of secondary antibodies, which comprise oligonucleotide barcodes for 1-3h, diluted in **Blocking buffer.** Then aspirate the secondary antibody and wash thoroughly 4x with **Complexing buffer.** Dilute the first and second label parts comprising suitably complementary barcode sequences configured to mediate attachment to the barcoded secondary antibodies in **Complexing buffer** and apply to sample. Allow the first and second label parts to hybridize to their respective secondary antibody. Typically, 5-60min at room temperature is sufficient. Proceed to step 8 to wash out unbound label parts.
8. Wash 4 × thoroughly with **Complexing buffer** each with agitation, even without nuclear staining, to remove unbound secondary antibody.
9. Optionally, perform a first readout and/or image the sample before applying the decomplexing condition. This readout/image may be used as a baseline and may improve the quality of the assay, but is not required in many cases.
10. Equilibrate the sample to the decomplexing condition by washing the sample 4x or flushing the sample with a suitable volume (e.g. 4-5 flow cell volumes) of **Decomplexing buffer.**
11. Allowing the hybridization of the first and second label parts to occur under the decomplexing condition, which may typically require 5-60min.
12. Optionally, provide a drop of mounting medium on a microscope slide and lay the coverslip with the cells or the biological sample upside down on this drop. Press the specimen with the tweezers slightly so that the mounting medium is well distributed, without squeezing the sample. The preparation is ready for microscopy after curing.
13. For optical-grade carrier bottom plates, dishes, or flow cells samples may be imaged directly in the **Decomplexing buffer,** in PBS, or any other imaging buffer of choice, so as long as the imaging buffer does not contain anything that disturbs the formed duplex between the first and second label parts.
14.Optionally, perform a comparison of the pixel intensities before and after allowing the duplex formation between the first and second label parts, i.e. under the complexing condition and decomplexing condition to calculate a fold change or ratio of the (FRET) signal on a pixel-per-pixel basis to assess the degree of proximity of the markers and thereby of the analytes.

### Example 3: Host-Guest controlled Proximity Hybridization Assay (HGPHA) on tissue sections:

The HGPHA for tissue sections is based on the multiplex IF protocol from Gerdes et al. 2013, Highly multiplexed single-cell analysis of formalin-fixed, paraffin-embedded cancer tissue, PNAS**.** While this is a robust protocol for cyclic immunofluorescence with FFPE tissue sections and tissue microarrays other protocols exist and may be equally suitable. To adapt them to the HGPHA assay one has to take care to perform the binding of first and second label part to the target under the complexing condition to avoid generation of false positives. The specific protocol steps then depend on whether the detection the user intend to use
A. Direct detection with directly labelled primary antibodies
B. Direct detection with barcoded primary antibodies
C. Indirect detection with directly labelled secondary antibodies
D. Indirect detection with barcoded secondary antibodies

The following example is provided for (A) and would have to be adapted accordingly to fit to the other options. The person skilled in the art will easily understand how to adapt the protocol to each of these options having read the description of the method provided herein and modify the protocol without deviating from the invention.

### Preparatory work:

(1) **Tissue biopsies** are collected according to standard procedures known to the skilled person and fixed with a fixative for example 4%PFA in PBS or formalin. The fixation buffer may further comprise detergents such as Tween 20, Triton X-100, saponin, or NP-40 in concentrations ranging from 0.01-1% depending on the tissue. Fixation time 1h to 3h at room temperature or overnight at 4°C.
(2) Tissues may then be **embedded** for cryosectioning or enter a workflow for paraffin embedding and sectioning, which involves stepwise dehydration.
(3) While **cryosections** may be directly used for downstream blocking, for Formalin-fixed paraffin-embedded **(FFPE)** sections **deparaffinization, rehydration, and epitope retrieval protocols** exists, which may also be applied to **tissue microarrays** (TMAs).

Note: The **Blocking buffer** used for tissue work contains a higher concentration of blocking agents than the one used for cells, likewise higher concentrations of detergent(s) may be used. For example, 10% (wt/vol) donkey serum and 3% (wt/vol) BSA).

### Main protocol:

(1) **Bake** formalin-fixed paraffin-embedded (FFPE) tissue samples or tissue arrays at 65 °C for 1 h.
(2) **Deparaffinize** with Histochoice clearing agent (Amresco) or comparable.
(3) **Rehydrate** by decreasing ethanol concentration washes **(ethanol series).**
(4) Perform **antigen retrieval** (Note: several established methods exist and are known to the person skilled in the art).
(5) Incubate in PBS with 0.3% Triton X-100 for 10 min at room temperature (Note: other **permeabilization** conditions may be equally suited or better suited depending on tissue type).
(6) Blocking with **Blocking Buffer** for 45 min at room temperature.
(7) Dilute oligonucleotide-barcoded primary antibodies in **Blocking Buffer** to optimized concentrations (typical range 0.1-10 µg/mL) and apply for 1-3h at room temperature or overnight at 4 °C.
(8) Wash thoroughly 4x each with agitation.
(9) Wash 4 × thoroughly to remove unbound primary antibody with PBS or **Blocking buffer** each with agitation.
(10) Dilute first and second label parts comprising complementary barcode sequences (i.e. complementary to the barcodes on the primary antibodies) in **Complexing buffer.**
(11) Apply first and second label parts diluted in **Complexing buffer** to the sample and allow to hybridize to the respective barcodes on the target-bound primary antibodies. This step may be carried out at room temperature for 5-60min or at a higher temperature with agitation.
(12) Wash 4x with **Complexing buffer** each with agitation to remove unbound first and second label parts. During this step one may also include DAPI for nuclear staining.
(13) Optionally, perform a first readout and/or image the sample before applying the decomplexing condition. This readout/image may be used as a baseline and may improve the quality of the assay but is not required in many cases.
(14) Equilibrate the sample to the decomplexing condition by washing the sample 4x each with agitation or flushing the sample with a suitable volume (e.g. 4-5 flow cell volumes) of **Decomplexing buffer.**
(15) Allowing the hybridization of the first and second label parts to occur under the decomplexing condition, which may typically require 5-60min.
(16) Optionally, provide a drop of mounting medium on a microscope slide and lay the coverslip with the cells or the biological sample upside down on this drop. Press the specimen with the tweezers slightly so that the mounting medium is well distributed, without squeezing the sample. The preparation is ready for microscopy after curing.
(17) For optical-grade carrier bottom plates, dishes, or flow cells samples may be imaged directly in the **Decomplexing buffer,** in PBS, or any other imaging buffer of choice, so as long as the imaging buffer does not contain anything that disturbs the formed duplex between the first and second label parts.
(18) Optionally, perform a comparison of the pixel intensities before and after allowing the duplex formation between the first and second label parts, i.e. under the complexing condition and decomplexing condition to calculate a fold change or ratio of the (FRET) signal on a pixel-per-pixel basis to assess the degree of proximity of the markers and thereby of the analytes.

While the examples provided are all microscopy or imaging examples, wherein a sample is imaged, it is clear that the invention may also be used for non-imaging detection. For example, the method may be used to detect the presence of two analytes on a cell like an immune cell, a bacterium, or a non-cellular particle like a virus or exosome. Thus, the method may be suited to analyse or sort cells or particles using flow cytometry and fluorescence activated cell sorting (FACS). Similarly, the readout may be performed by plate reading of the (FRET signal). Alternatively, the method may be configured for a lateral flow assay and be used to detect a pathogen or a hormone with very high specificity.

The method may further be carried out in a cyclical fashion, wherein a cycle consists of staining, reading out/imaging, and inactivation.

### Further remarks on the complexing and decomplexing condition

The nucleic backbone of the first and second label part may be preferably 20-120nt in length and comprise one or multiple guest molecules like 1-5 guest molecules for every 10nt (nt = nucleotides).

In a further aspect, a marker for analysing a biological sample with a plurality of target analytes is provided. The marker comprises a label as described above with a first label part and a second label part. The marker further comprises a first marker part comprising a first affinity reagent and the first label part, and a second marker part comprising a second affinity reagent and the second label part. The first affinity reagent and the second affinity reagent are each configured to bind specifically to one of the target analytes of the biological sample.

The marker enables efficiently determining the distance or proximity between two target analytes. The marker also enables precisely determining the presence and/or location of the two target analytes or of a single target analyte in a biological sample. The biological sample may be a cellular sample, such as a tissue section, for example. Further examples include biopsy samples such as liquid biopsy or a tissue biopsy.

The affinity reagents of the marker may be antibodies, antibody fragments, amino acid- or nucleic acid-based aptamers, or linear nucleic acids. This enables detecting a larger variety of target analytes. In particular, the nucleic acid backbone of a label part is attached to the respective affinity reagent, preferably covalently attached.

The affinity reagents of the marker may bind to a single target analyte or to two target analytes. In the first case, the first affinity reagent may be configured to bind specifically to a first area or epitope of the single target analyte and the second affinity reagent may be configured to bind specifically to a second area or epitope of the single target analyte. By requiring two marker parts to bind to the single target analyte, the presence and/or location of the single target analyte may be precisely determined, in particular with an increased specificity. In the second case, the first affinity reagent may be configured to bind specifically to a first target analyte and the second affinity reagent may be configured to bind specifically to a second target analyte. This enables determining the distance or proximity between the first target analyte and the second target analyte.

Preferably, the marker comprises an anchor oligonucleotide configured to anchor the marker to a solid support. This enables use of the marker in a flow through assay, for example. In particular, the anchor oligonucleotide may be (covalently) attached to the first affinity reagent or the second affinity reagent. The anchor oligonucleotide may comprise an affinity moiety configured to specifically bind to a respective affinity moiety arranged on the solid support.

In another aspect, a method for analysing a biological sample is provided. The method comprising introducing at least one marker, as described above, into the biological sample. The step of introducing the at least one marker may optionally include waiting for a set amount of time to allow binding of the at least one marker to a respective target analyte of the biological sample. After introducing the at least one marker, any marker not bound to the target analyte may optionally be removed from the biological sample, for example by washing. Further optionally, at least during introducing the at least one marker and removing the unbound markers, a complexing condition is applied to the biological sample. The complexing condition enables the formation of a complex between the guest molecule and the host molecule and thereby avoid hybridisation of the first and second nucleic acid backbones of the marker. The method further includes applying a decomplexing condition to the biological sample, in particular after introducing the at least one marker into the biological sample. The decomplexing condition may include adjusting a salt concentration, adjusting temperature, and/or addition of a competitive guest molecule that is not attached to the marker, for example. This decomplexes or removes the guest-host complex and allows hybridisation of the first and second nucleic acids of the marker parts. The method further includes generating an optical readout of the biological sample with the marker, for example by means of a microscope, such as a fluorescence microscope.

In particular, the at least one marker introduced into the biological sample includes the at least one host molecule. Thus, the complex with the guest molecule may be formed. The step of introducing the at least one marker may include adding individual parts of the at least one marker to the sample separately, in particular separately over time.

Generally, a plurality of markers may be introduced into the biological sample, the markers being specific to respective target analytes or pairs of target analytes, in order to identify a large number of (different or the same) target analytes at the same time.

Preferably, a target analyte is identified and/or localised within the biological sample based on the label(s) of the marker(s), in particular the labelling moieties, associated with the target analyte in the optical readout. Markers may be physically constituted before introducing them into the biological sample. Alternatively, affinity reagents may be barcoded with oligonucleotide barcodes and introduced into the biological sample initially and the labels comprising complementary barcode oligonucleotides may be introduced into the biological sample in a subsequent step and may then attach to their respective affinity reagent thereby forming the respective markers within the biological sample.

In another aspect, a kit for analysing a biological sample comprising a marker, as described above, and at least one host molecule configured to form a complex with the at least one guest molecule of the marker is provided. In another aspect, a use of a label, as described above, or a use of a marker, as described above, or a use of a kit, as described above, for a proximity assay for analysing a biological sample is possible.

The marker, kit and the method and the use of the method have the same advantages as the label. Further, the marker, kit and the method may be supplemented with the features of the label described in this document, in particular, the features of the dependent claims of the label.

### Short Description of the Figures

Hereinafter, specific embodiments are described referring to the drawings, wherein:
- Figure 1: is a schematic view of a first and a second nucleic acid backbone with a host and a guest molecule,
- Figure 2: is a schematic view of a marker for analysing a biological sample,
- Figure 3: is a schematic view of the marker according to Fig. 2,
- Figure 4: is a schematic view of the marker according to Fig. 2,
- Figure 5: is a schematic view of a first label part and second label part,
- Figure 6: is a schematic view of a first label part and second label part, according to a second embodiment,
- Figure 7: is a schematic view of a marker according to a second embodiment under a complexing condition,
- Figure 8: is a schematic view of the marker according to Figure 7 under a decomplexing condition,
- Figure 9: is a schematic view of several markers according to further embodiments under complexing and decomplexing conditions,
- Figure 10: is a schematic view of two markers comprising secondary affinity reagents,
- Figure 11: is a flow chart of a method for amplifying barcode oligonucleotides of markers,
- Figure 12: is a schematic view of a marker comprising a dendrimeric oligonucleotide-based structures configured to bind multiplicities of first and second label parts,
- Figure 13: is a schematic view of an hgPHA assay microarray spotted on a solid support under a complexing condition,
- Figure 14: is a schematic view of an hgPHA assay microarray according to Figure 13 under a decomplexing condition,
- Figure 15: is a schematic view of an hgPHA assay used in conjunction with a capture (micro)array spotted on a solid support under a complexing condition,
- Figure 16: is a schematic view of an hgPHA assay according to Figure 15 under a decomplexing condition,
- Figure 17: is a schematic view of a marker attached to a solid support,
- Figure 18: is a schematic view of a marker with a tertiary antibody attached to a solid support,
- Figure 19: is a schematic view of a marker with a tertiary antibody attached to a solid support according to a second embodiment,
- Figure 20: is a schematic view of a marker with a tertiary antibody attached to a solid support according to a third embodiment,
- Figure 21: is a schematic view of a cellular sample and a marker used to determine the proximity between two cells of the cellular sample, and
- Figure 22: is a flow chart of a method for analysing a sample with a marker.

### Detailed Description

Figure 1 is a schematic view of a first nucleic acid backbone 100 and a second nucleic acid backbone 102, for example of a label for analysing a biological sample. The first nucleic acid backbone 100 comprises a guest molecule 104. The first nucleic acid backbone 100 and the second nucleic acid backbone 102 each are polynucleotides. The first nucleic acid backbone 100 and the second nucleic acid backbone 102 are at least partially complementary to each other. This enables hybridisation of the first nucleic acid backbone 100 and the second nucleic acid backbone 102, as shown on the right side of Fig. 1.

Either one of or both of the first nucleic acid backbone 100 and the second nucleic acid backbone 102 may comprise a nucleic acid analogue such as a xeno nucleic acid (XNA), a peptide nucleic acid (PNA), a locked nucleic acid (LNA), a morpholino (MO), a phosphorothioate-modified DNA (PT-DNA). Alternatively, one or both of the first nucleic acid backbone 100 and the second nucleic acid backbone 102 may be a naturally occurring nucleic acid. Either one of or both of the first nucleic acid backbone 100 and the second nucleic acid backbone 102 may further comprise modified nucleobases.

The guest molecule 104 is covalently attached to one of the nucleotides (indicated as "N") of the first nucleic acid backbone 100 (and/or the second nucleic acid backbone 102). The conjugation of the guest molecule may be as reported in Xiao et al. 2022 we *"introduced the guest molecules into the 4'-amino position of cytosine and the 6'-amino position of adenine".* As depicted in Figure 1 of Xiao et al. 2022 this means that the modified bases may still base pair. The guest molecules 104 and/or the ratio of guest molecules 104 to the number of nucleotides of the first nucleic acid backbone 100 or the second nucleic acid backbone 102 are preferably chosen such that the first nucleic acid backbone 100 and the second nucleic acid backbone 102 can hybridise to each other. For example, one guest molecule is provided for every 5 to 10 nucleotides of the respective nucleic acid backbone. The length of the nucleic acid backbones, the number of guest molecules, the type and thereby affinity of the guest molecules to the host molecule, as well as the buffer compositions of complexing and decomplexing buffers (e.g. salt, formamide, dextran, blocking nucleic acid concentration) may each be modified to tune the melting temperatures of the nucleic backbones under the complexing (Tmc) and decomplexing (Tmdc) condition. This may be done in such a way that under room temperature the nucleic acid backbones hybridize only under the decomplexing condition. Alternatively, the complexing condition may comprise a higher temperature than the decomplexing condition. For example, binding of the first and second label part may be performed at 30°C, 37°C, 50-65°C, or even at higher temperatures. The selection of a suitable temperature for this step will also depend on the specific application and type of sample. For immunofluorescence in tissue sections for example it is desirable to stay well below 60°C to avoid the formation of fluorescence background. In other assays, for example with liquid biopsies or lysates this may not be an issue and hence higher temperatures may be acceptable. In most assays the hybridised first and second nucleic acid backbones may have a melting temperature in the range of 45-65°C, and/or 65-90°C.

In this regards, it is clear that following the binding of the primary affinity reagents to their targets, for example the primary antibodies to their respective target analytes, it is possible to cross-link said affinity reagents with the analytes or other proteins in the sample. Alternatively, or in addition the sample may be infused with polymer components such as acrylamide and a gel may be formed in the sample. Such gels may carry reactive groups that allow crosslinking and may also facilitate expansion of the sample. The person skilled in the art will know such techniques from the field of expansion microscopy and may easily expand and/or modify the method provided herein accordingly without deviating from the invention.

On the left side of Fig. 1 a complexing condition, in particular a high concentration of a host molecule 106, is provided. The guest molecule 104 and the host molecule 106 are configured to form a guest-host complex 108. This guest-host complex 108 reduces the melting temperature, preferably to/by about 10°C for each incorporated guest molecule for very high-affinity guests such as adamantane (AD) or adamantane-derivatives. For ferrocene (FC) Xiao et al (2022). reported about a ΔTm -9.6°C for CB[7] concentrations ranging from 1-300µM for an 15nt oligonucleotide with a single modified nt. For ferrocene (FC) the ΔTm 0 to -8.5°C for the same sequence in the range of 1-300µM. A further 19nt oligonucleotide with two modified guest modified nucleobases had a Tm of 55.2 (AD) and 55.5 (FC) in the absence of CB[7], which lowered to 30.5°C (AD) and 37°C (FC) respectively at 300µM. This example provides a good indication for useful lengths of the first and second nucleic acid backbone, useful number of guest molecules per length in nt, as well the differences in melting temperatures that are to be expected under complexing and decomplexing conditions.

The presence of the host molecule 106 results in the dissociation of the first nucleic acid backbone 100 and the second nucleic acid backbone 102 or it hinders an initial hybridisation of the first nucleic acid backbone 100 and the second nucleic acid backbone 102.

On the right side of Fig. 1 a decomplexing condition, in particular a low concentration of the host molecule 106, is provided. As described above, this allows hybridisation of the first nucleic acid backbone 100 and the second nucleic acid backbone 102.

The interaction between guest molecules and host molecules is a fundamental concept in supramolecular chemistry, where the host molecules provide a structural framework that encapsulates or binds the guest molecules through non-covalent interactions. These interactions can include hydrogen bonding, van der Waals forces, electrostatic interactions, and hydrophobic effects. The specificity and strength of the interaction depend on the complementarity between the host and guest in terms of shape, size, and functional groups. For example, cyclodextrins may act as host molecules that can form inclusion complexes with a variety of guest molecules, such as aromatic compounds, and fatty acids.

Another example is cucurbit[n]urils, a family of macrocyclic molecules, which can encapsulate guests ranging from small metal ions to large organic molecules like drugs and dyes, forming highly stable complexes. Examples of small organic molecules that form stable complexes with cucurbit[7]urils include adamantane and adamantane derivatives (AD) and ferrocene and ferrocene derivatives (FC). Examples of aromatic compounds that form stable complexes with cucurbit[7]urils include methyl viologen and 1,4-dimethoxybenzene. Specific examples of suitable guest molecules include 1-adamantanemethylamine (AD), ferrocenyl methylamine (FC), 1,4-benzenedimethanamine (BA), and 4-tertbutylbenzylamine (TB) as reported by Xiao et al. 2022.lt is important to note that the affinities of these guest molecules, when conjugated to DNA may change as further reported by Xiao et al. 2022, who found by isothermal titration calorimetry (ITC) analysis that *"CB[7] still specifically bound with the AD (Kₐ = 1.31 × 10⁷ M⁻¹) and FC (Kₐ = 2.95* × *10⁶ M⁻¹) groups in the context of DNA strand but exhibited feeble affinities on the BA and TB groups".* The impact on duplex stability varies strongly with the affinities as further described in Xiao et al. and summarized in the Table 1 therein. Further suitable guest molecules may be found using modern molecular docking approaches.

By applying the complexing condition or the decomplexing condition to the host and guest molecules 104, 106, the association or dissociation of the host and guest molecules 104, 106 can be effected. In turn, this enables causing the first and second nucleic acid backbone 100, 102 to hybridise and form a duplex or to melt and dissociate.

An example of the decomplexing condition may be the addition of a competitive guest molecule, which causes the host molecule 106 that is complexed with the guest molecule 104 to form a complex with the competitive guest molecule instead. A competitive guest molecule may be of the same molecular species (only in the free form) or a different one. In this regard, one could use FC as a guest molecule to modify the first and/or second nucleic acid backbone, which would require relatively high concentrations of host molecule as detailed above in the complexing condition like ^{~}100-300µM and then use AD as a competitive guest molecule.

Further examples of parameters that cause complexing and decomplexing or can influence the affinity of the guest and host molecule 104, 106 is salt concentration, pH, and temperature.

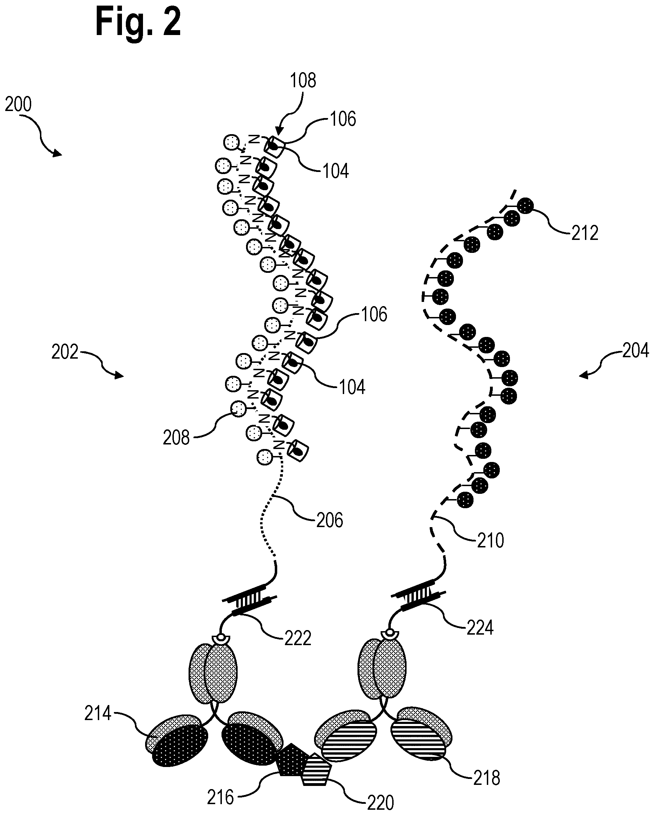

Figure 2 is a schematic view of a marker 200 for analysing a biological sample. The marker 200 comprises a first marker part 202 and a second marker part 204. The first marker part 202 may comprise a first label part with a first nucleic acid backbone 206, a plurality of first labelling moieties 208, and a plurality of guest molecules 104. The first labelling moieties 208 and the guest molecules 104 may be attached to the first nucleic acid backbone 206. In addition, a plurality of host molecules 106 are provided. Each guest molecule 104 forms a complex 108 with one of the host molecules 106.

The second marker part 204 comprises a second label part with a second nucleic acid backbone 210, and a plurality of second labelling moieties 212 attached to the second nucleic acid backbone 210.

The first nucleic acid backbone 206 and the second nucleic acid backbone 210 are at least partially complementary to each other. This enables hybridisation of the first nucleic acid backbone 206 and the second nucleic acid backbone 210. In Fig. 2 the marker 200 is shown together with the host molecules 106. The resulting host-guest complex 108 prevents the hybridisation of the first nucleic acid backbone 206 and the second nucleic acid backbone 210.

The first marker part 202 further comprises a first affinity reagent 214 configured to specifically bind to a first target analyte 216. Similarly, the second marker part 204 further comprises a second affinity reagent 218 configured to specifically bind to a second target analyte 220.

The first label part 202 is attached to the first affinity reagent 214 via a barcode oligonucleotide 222 of the first affinity reagent 214. Similarly, the second label part 204 is attached to the second affinity reagent 218 via a barcode oligonucleotide 224. Each barcode oligonucleotide 222, 224 is preferably specific to the respective affinity reagent 214, 218 and/or the respective label part 202, 204. In particular, the first and second affinity reagent 214, 218 are antibodies.

The first marker part 202 is bound to the first target analyte 216 via the first affinity reagent 214. Similarly, the second marker part 204 is bound to the second target analyte 220 via the second affinity reagent 218.

In the exemplary embodiment of Fig. 2, the target analytes 216, 220 are in contact, for example, due to an affinity interaction between the target analytes 216, 220. This results in the marker parts 202, 204 to be in proximity once they are bound to the target analytes 216, 220. As described above, despite their proximity, the first nucleic acid backbone 206 and the second nucleic acid backbone 210 do not hybridise to each other due to the host-guest complex 108. This enables easy handling of the marker 200, in particular its marker parts 202, 204 during an analysis of a biological sample containing the target analytes 216, 218. For example, in case the marker parts 202, 204 are not yet bound to the target analytes 216, 218 the host-guest complex 108 prevents the undesired uncontrolled hybridisation of the first nucleic acid backbone 206 and the second nucleic acid backbone 210.

Additionally or alternatively, guest molecules 104 may be attached to the second nucleic acid backbone 210.

The first labelling moieties 208 and the second labelling moieties 212 may be fluorophores that act as FRET donors or FRET acceptors, respectively. Thus, each first labelling moiety 208 may form a FRET pair with one of the second labelling moieties 212. Since the first nucleic acid backbone 206 and the second nucleic acid backbone 210 are not hybridised in the state shown in Fig. 2, the first nucleic acid backbone 206 and the second nucleic acid backbone 210 are not in close proximity to each other. In particular, the first labelling moieties 208 and the second labelling moieties 212 are not within a distance that enables a FRET to occur.

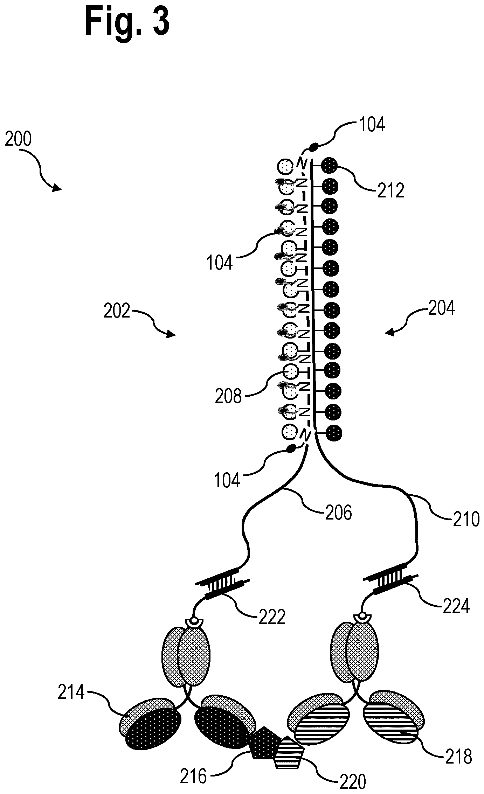

Figure 3 is a schematic view of the marker 200 without the host molecules 106 present. Thus, no host-guest complex 108 is formed in the state shown in Fig. 3. For simplicity, only two exemplary guest molecules 104 are shown in Fig. 3.

Without the presence of the host molecules 106, the first nucleic acid backbone 206 and the second nucleic acid backbone 210 can hybridise to each other, as shown in Fig. 3. The hybridisation of the first nucleic acid backbone 206 and the second nucleic acid backbone 210 brings the first labelling moieties 208 and the second labelling moieties 212 into proximity such that a FRET may occur between the first labelling moieties 208 and the second labelling moieties 212. Thus, the proximity of the target analytes 216, 220 may be determined by the occurrence of FRET between the first labelling moieties 208 and the second labelling moieties 212, for example, by means of a fluorescence microscope. In Fig. 3, a FRET can occur between all the first labelling moieties 208 and the second labelling moieties 212.

In a method for analysing a biological sample, the marker 200, in particular the marker parts 202, 204, may initially be introduced into the biological sample under a complexing condition with the host molecule 106 present and the guest-host complex 108 formed, as shown in Fig. 2. After waiting for an appropriate amount of time for the marker parts 202, 204 to bind, any marker parts 202, 204 that remain unbound to a respective target analyte 216, 218 may be removed, for example by washing the biological sample. The steps described above are preferably carried out under the complexing condition.

Subsequently, a decomplexing condition may be applied, which results in the decomplexing of the guest-host complex 108. In particular, this results in the removal of the host molecule 106 from the marker 200. The decomplexing condition may include a change in temperature to decomplex the guest-host complex 108 and a subsequent removal of the host molecule 106 by washing the biological sample, for example. Alternatively or in addition, a competitive guest molecule may be introduced into the biological sample. Fig. 3 shows the marker 200 after removal of the host molecule 106.

The removal of the host molecule 106 enables hybridisation of the first nucleic acid backbone 206 with the second nucleic acid backbone 210. Subsequently, an optical readout may be generated of the biological sample with the marker 200. The optical readout may be an image, for example, generated by means of a fluorescence microscope.

The optical readout enables determining whether or not the target analytes 216, 220 are in close proximity to each other. In particular, the optical readout can be used to determine whether or not a FRET occurs between the first labelling moieties 208 and the second labelling moieties 212, by observing a respective change in their optical properties due to the FRET. Additionally, it can be determined by at least one suitable optical readout if and where unbound FRET pairs, i.e. first labelling moieties 208 and the second labelling moieties 212 not being located closely together, are bound to target analytes in the biological sample. This can be done e.g. by exciting the labelling moieties acting as a donor with suitable excitation light and determine if these labelling moieties excite intrinsic fluorescence light according to their intrinsic characteristic. A further readout can be made by exciting the labelling moieties acting as an acceptor with suitable excitation light and determining, if these labelling moieties excite intrinsic fluorescence light according to their intrinsic characteristic.

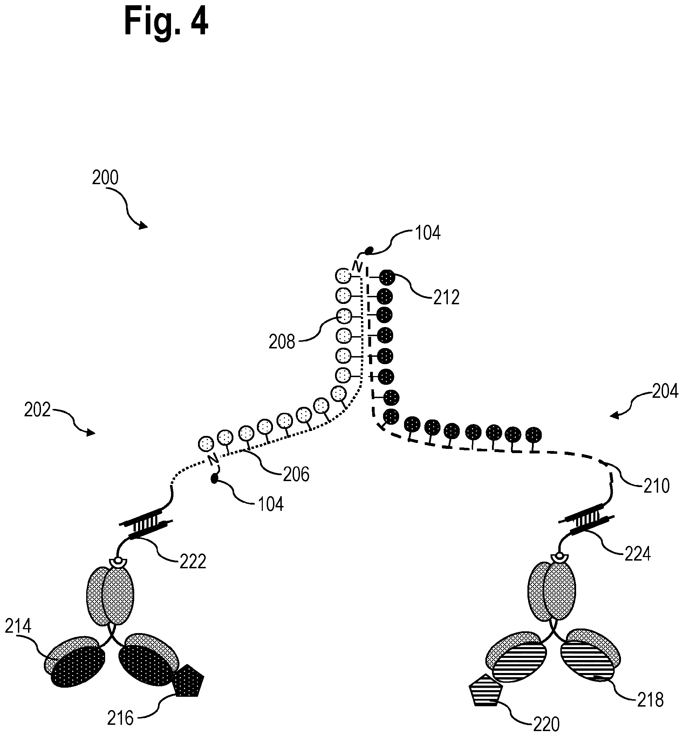

Figure 4 is a schematic view of the marker 200 with the target analytes 216, 220 at a greater distance from each other compared to the Figs. 2 and 3. Similarly to Fig. 3, the host molecule 106 is not present and the first nucleic acid backbone 206 and the second nucleic acid backbone 210 can hybridise to each other. However, the greater distance between the target analytes 216, 220 results in the first nucleic acid backbone 206 and the second nucleic acid backbone 210 to only partially hybridise, in particular to hybridise to a lesser extent compared to the state shown in Fig. 3, in which the target analytes 216, 220 are in contact with each other. For simplicity, only two exemplary guest molecules 104 are shown in Fig. 4. The marker 200 may comprise more guest molecules 104.

The partially hybridised first nucleic acid backbone 206 and second nucleic acid backbone 210 results in some of the first labelling moieties 208 and the second labelling moieties 212 to be at a greater distance from each other. In particular, these first labelling moieties 208 and second labelling moieties 212 may at a distance from each other, at which no FRET occurs between them. Thus, the overall observable FRET efficiency is reduced compared to the state shown in Fig. 3. This reduced FRET efficiency can be observed when generating an optical readout of the biological sample with the marker 200.

In summary and with particular reference to Figs. 3 and 4, it can be seen that the distance between the target analytes 216, 220 has a direct effect on the degree of hybridisation between the first nucleic acid backbone 206 and second nucleic acid backbone 210 and therefore on the overall FRET efficiency between the first labelling moieties 208 and second labelling moieties 212. The FRET efficiency between the first labelling moieties 208 and second labelling moieties 212 is therefore proportional to the distance between the target analytes 216, 220 and can be used to determine the distance or proximity between the target analytes 216, 220.

Thus, the spatial stringency of the assay depends on the size of the affinity reagents in particular the distance between the paratope:epitope interface or more generally the site where target analyte and affinity reagent bind to each other to the site where the first or second label part is coupled to (coupling site). Further the spatial stringency depends on linkers and/or barcode oligonucleotides as well as the length of the first and second label part. For this reason, the spatial stringency of a hgPHA assay is tuneable over a wide range, e.g. few nanometres to hundreds of nanometres. For very low spatial stringency assay, which may be used to detect cellular proximities in a tissue, the first and second label part may further comprise a non-nucleic acid linker like a PEG linker for example.

Figure 5 is a schematic view of a first label part 500 and a second label part 502 that can be used for a FRET-based hgPHA assay. The first and second label parts 500, 502 each comprise at least partially complementary nucleic acid backbone 504, 506, which are shown as dotted 504 and dashed 506 lines in Figure 5. For a FRET-based hgPHA assay, the first label part 500 is conjugated to one of a FRET donor 508 and FRET acceptor 510 and the second label part 502 is conjugated to the other one of a FRET donor 508 and FRET acceptor 510. Suitable FRET pairs are described in the section on FRET above.

The first label part 500 further comprises guest molecules 104. In a configuration indicated with reference sign 500a, the first label part 500, in particular the guest molecules 104, is decomplexed from the host molecules 106. In the configuration indicated with reference sign 500b, the first label part 500, in particular the guest molecules 104, is complexed with the host molecules 106.

In the following Figures, the first label part 500 and the second label part 502 may be shown schematically by the respective half annulus indicated in Fig. 5.

As an alternative to or in addition to FRET as a mechanism for allowing the detection of the hybridization one can also setup hgPHA assays based on dequenching of strongly quenched labels.

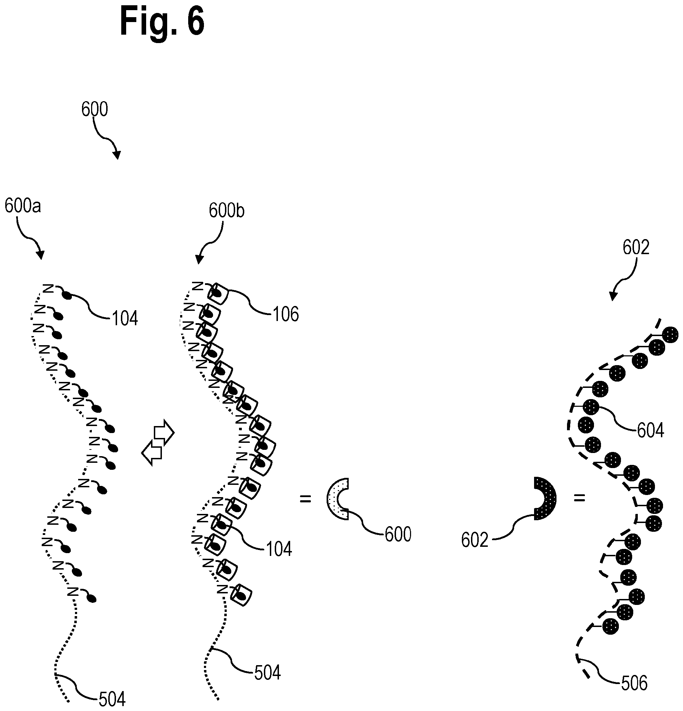

Figure 6, for example, is a schematic view of a first label part 600 and a second label part 602. The first and second label parts 600, 602 each comprise at least partially complementary nucleic acid backbones 504, 506. The first label part 600 does not comprise a labelling moiety. However, the second label part 602 is conjugated to strongly (self-)quenching labelling moieties 604, such as ATTO647N or other hydrophobic dyes. These hydrophobic dyes, when conjugated to the single-stranded DNA-backbone 506 that exhibits high flexibility, are free to aggregate and quench each other. This quenching reduces the emission of the labelling moieties 604 of the second label part 602 strongly. For example, a ~120nt ssDNA oligonucleotide-based label comprising 10 ATTO647N dye molecules will exhibit about the same or less emission than a single ATTO647N dye molecule. The duplex formation of such a single-stranded label part 602 with the complementary strand 504 of the first label part 600 leads to a strong increase in fluorescence emission, which is easily detectable. This is further described in the European patent application with the application number 24177155.9, the complete content thereof being incorporated herein by reference. Examples of hydrophobic labelling moieties that are regularly used to analyse biological samples include ATTO 390, ATTO 425, ATTO 550, ATTO Rho12, ATTO 633, and ATTO 647N. In particular, the dyes mentioned above may exhibit undesired aggregation when multimerized on a single stranded DNA nucleic acid backbone with a dye-to-dye distance of 8 nt.

The first label part 600 further comprises guest molecules 104. In a configuration indicated with reference sign 600a, the first label part 600, in particular the guest molecules 104, is decomplexed from the host molecules 106. In the configuration indicated with reference sign 600b, the first label part 600, in particular the guest molecules 104, is complexed with the host molecules 106.

Similar to the label parts 500, 502, the label part 600, 602 may be shown schematically in subsequent Figures by the respective half annulus indicated in Fig. 6.

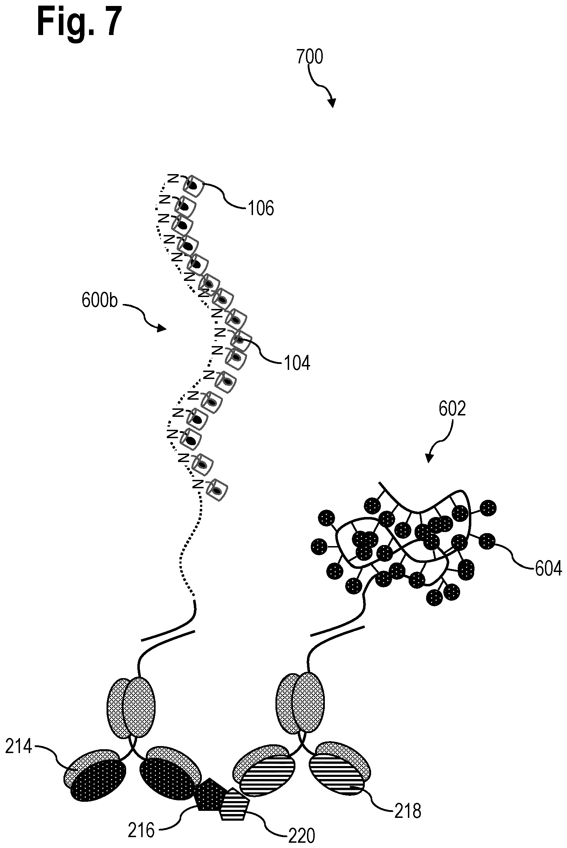

Figure 7 is a schematic view of a dequenching-based hgPHA assay according to another embodiment comprising a marker 700. The marker 700 comprises the first and second label parts 600, 602, which are in close proximity, as the affinity reagents 214, 218 are bound to their respective target analytes 216, 220 that interact with each other. Under a complexing condition the first label part 600 comprising the guest molecules 104 is complexated fully or at least to a substantial degree with host molecules 106 e.g. CB[7], which inhibits the hybridization to the complementary second label part 602. The second label part 602 comprises a multiplicity of hydrophobic or moderately hydrophobic dye molecules 604 e.g. ATTO 390, ATTO 425, ATTO 550, ATTO Rho12, ATTO 633, ATTO 647N, ATTO 680, and ATTO 700. Such hydrophobic or moderately hydrophilic dye molecules have a tendency to aggregate and self-quench, when multimerized on a polymeric backbone such as the nucleic acid backbone 506 of the second label part 602. As described in the European patent application with the application number 24177155.9, this self-quenching on ssDNA backbones can be avoided by forming a duplex or double stranded nucleic acid complex. For a dequenching-based hgPHA assay this behaviour is exploited as a sensing mechanism. Only in the presence of the first label part 600 and the second label part 602, i.e. only when they are present and in close proximity and only under the decomplexing condition the duplex can be formed, which leads to reduction in flexibility or increase in persistence length of the nucleic acid backbone duplex.

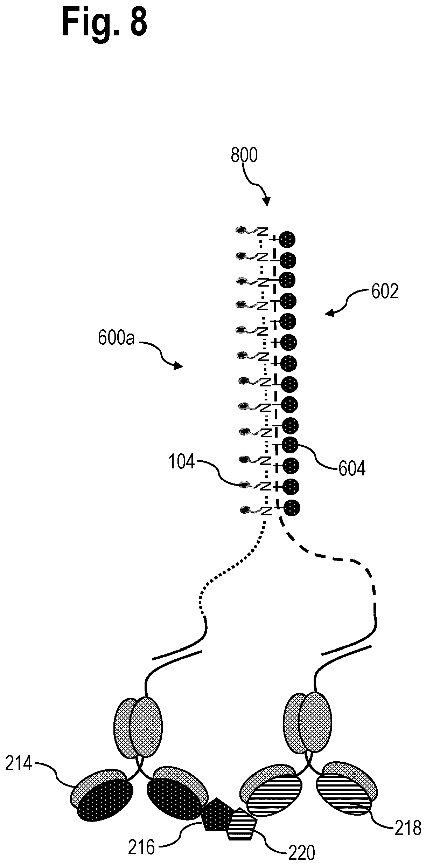

Figure 8 shows the marker 700 with the duplex 800 formed between the label parts 600, 602. The corresponding increase in persistence length reduces the ability for dye-dye interaction and self-quenching. In a particularly preferred embodiment, the first label part 600 is around 100-120nt in length and comprises 5-20 guest molecules 104 and the second label part 602 is around 100-120nt in length and comprises 5-20 dye molecules 604. For example, dye molecules may be arranged at a distance of 5-15nt, more preferably of 8nts. Using dequenching as a detection mechanism for hybridization or duplex formation between the first label part 600 and the second label part 602, provides the particular advantage that only one label part, for example the second label part 602, has to be conjugated to a dye molecule 604. This facilitates setting up hgPHA multiplex assay, in which multiple colours are used, e.g. different second label parts 602 may comprise different fluorescent dyes. A range of hydrophobic to moderately hydrophilic dyes are well suited to setup dequenching-based hgPHA assays. Aside from this it also reduces the cost of the assay. It is worth noting that FRET- and Dequenching may also be combined in one assay.

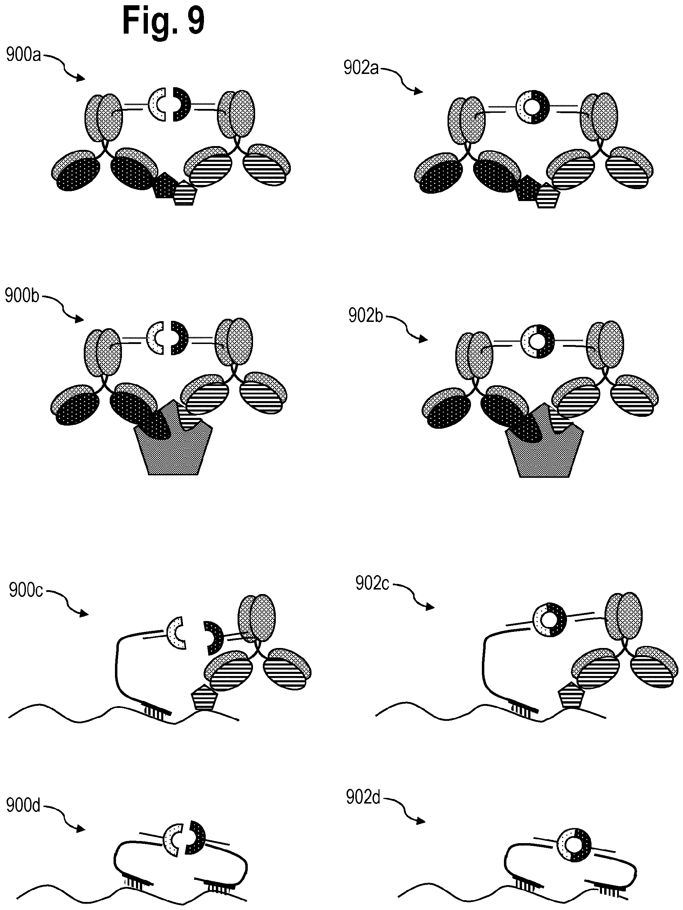

Figure 9 is a schematic overview of various hgPHA formats. The left-hand side shows exemplary markers 900a, 900b, 900c, 900d bound to target analytes under a complexing condition. The right-hand side shows respective markers 902a, 902b, 902c, 902d under a decomplexing condition. The label parts of the markers 900a, 900b, 900c, 900d, 902a, 902b, 902c, 902d are shown by the half annulus symbols indicated in Figs. 5 and 6.

The first row shows the marker 900a, 902a suitable for detecting protein-protein proximity as a measure for protein-protein interaction. In this example two antibodies are shown, but other protein-binding affinity reagents like affimers, aptamers, nanobodies, single-domain antibodies are equally suited. Likewise, in addition to proteins the target analytes may also be metabolites or any other analyte class for examples.

The second row shows the marker 900b, 902b comprising an antibody pair or more used to detect a single target analyte. This may be used to increase specificity or to interrogate, posttranslational modifications, alternative splicing, or proteolytic processing/degradation.

The third row shows the marker 900c, 902c comprising a protein binding affinity reagent (e.g. antibody) and the other comprises a nucleic acid target binding affinity reagent (e.g. oligonucleotide probe). Such a marker may be used to detect for example RNA-protein interaction or DNA-protein interaction and may be used to study the binding of transcription factors and their impact on gene regulation, the binding or modification of histones at a certain locus, as well as may be used to assess the integration site of genetic modifications. The latter is particularly relevant to development and quality control in cell and gene therapy. Furthermore, this format lends itself well to be used with CRISPR and similar systems to assess target binding.

The fourth row shows the marker 900d, 902d for detecting nucleic acid targets and may be used to detect mRNA with high specificity.

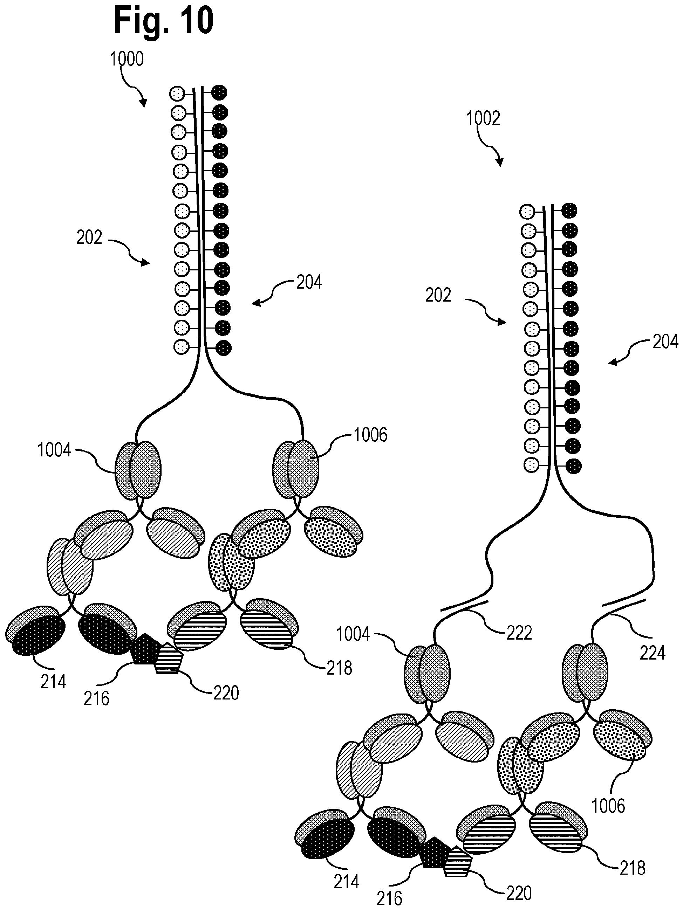

Figure 10 is a schematic showing two markers 1000, 1002 that are based on modified indirect immunofluorescence. The markers 1000, 1002 comprise the label parts 202, 204, pairs of primary antibodies 214, 218, which are configured to bind to the target analytes 216, 220 in a biological sample, and secondary antibodies 1004, 1006. The secondary antibodies 1004, 1006 may be bind specifically to a particular one of the primary antibodies 214, 218. The label parts 202, 204 may be directly conjugated to the secondary antibodies 1004, 1006, in case of marker 1000. Alternatively, in case of marker 1002, the label parts 202, 204 may be indirectly conjugated to the secondary antibodies 1004, 1006, for example via hybridisation to respective barcode oligonucleotides 222, 224. The use of barcoded affinity reagents is many times desirable as it enables antibody binding (either or both primary and secondary antibodies) under a decomplexing condition, which may be buffers usually used in immunofluorescence protocols for washing, antibody binding, or blocking. The first and second label parts 202, 204 can in this case be applied to the sample in a separate step under a complexing condition.

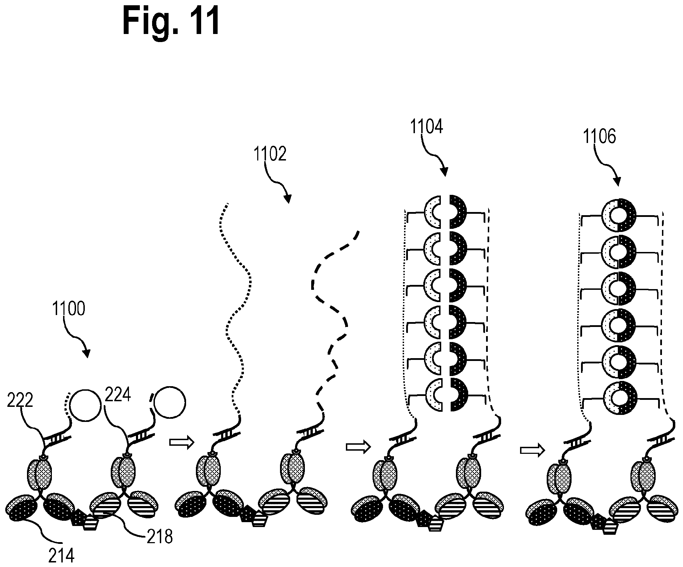

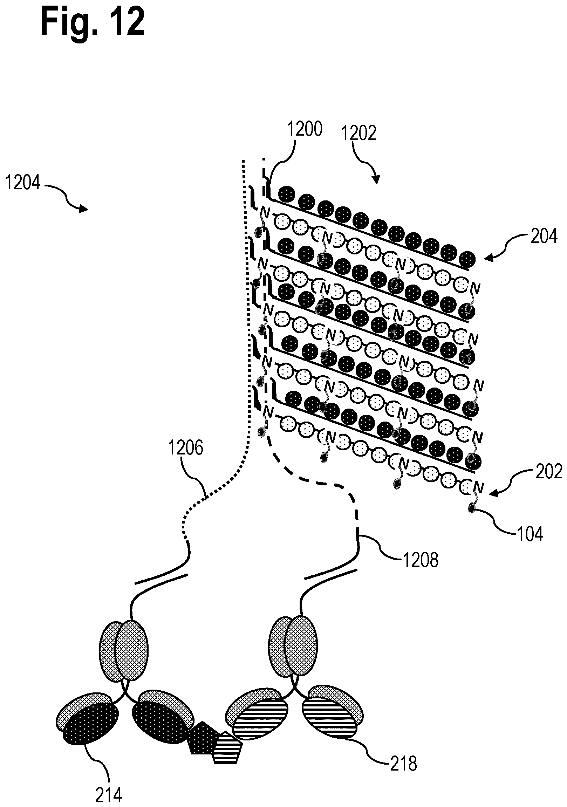

Further, as shown in the schematic workflow in Figure 11 the use of barcoded affinity reagents 214, 218 both primary and secondary allows barcode 222, 224 amplification via enzymatic methods including loop-mediated isothermal amplification (LAMP), rolling circle amplification (RCA), polymerase chain reaction (PCR), or non-enzymatic reactions like hybridization chain reaction (HCR), primer-exchange reaction (PER), or via the use of additional oligonucleotides such as "L-adapters" 1200 (see Fig. 12) that allow amplification by way of generation of dendrimeric structures 1202, or by way of attaching DNA-nanostructures such as DNA origamis, or by in situ generation of DNA-nanostructures such as DNA-brick-based or similar structures. Thus, barcode oligonucleotides 222, 224 can be amplified in situ to generate a plurality of attachment sites for hybridising label parts, in particular their respective backbones.

The workflow may comprise the following steps:
Step 1100: Introducing oligonucleotide barcoded affinity reagents (AR) and allowing them to bind to target analytes. And washing out unbound AR.
Step 1102: Optionally, amplifying barcode sequences using e.g. LAMP or RCA; Optionally, amplification may be achieved by building dendrimeric DNA-based structures or hybridization chain reaction (HCR).
Step 1104: Hybridizing first and second label parts to amplified barcode sequences avoiding the hybridization of the first and second label part(s) to each other by applying the complexing condition. Optionally, performing a "baseline" readout/imaging.
Step 1106: Allowing the hybridization of the first and second nucleic acid backbone of the first and second label part by applying decomplexing condition. Washing out unbound label parts. Performing, readout/imaging.

Optionally, after step 1106, label parts may be removed by applying an inactivation or removal agent, e.g. DNase I, and repeating the process for by starting with step 1100 again.

Figure 12 shows schematically an example for how "L-adapter" oligonucleotides 1200 may be used to build dendrimeric structures 1202. To that end, the marker 1204 shown in Figure 12 comprises a first marker part with a first attachment oligonucleotide 1206 that comprises a plurality of attachment sequences, to which the backbone 206 of the first label part 202 is partially complementary to. Similarly, the marker 1204 comprises a second marker part with a second attachment oligonucleotide 1208 that comprises a plurality of attachment sequences, to which the backbone 210 of the second label part 204 is partially complementary to. Thus, a plurality of first and second label parts 202, 204 may hybridise to the respective attachment oligonucleotides 1206, 1208.

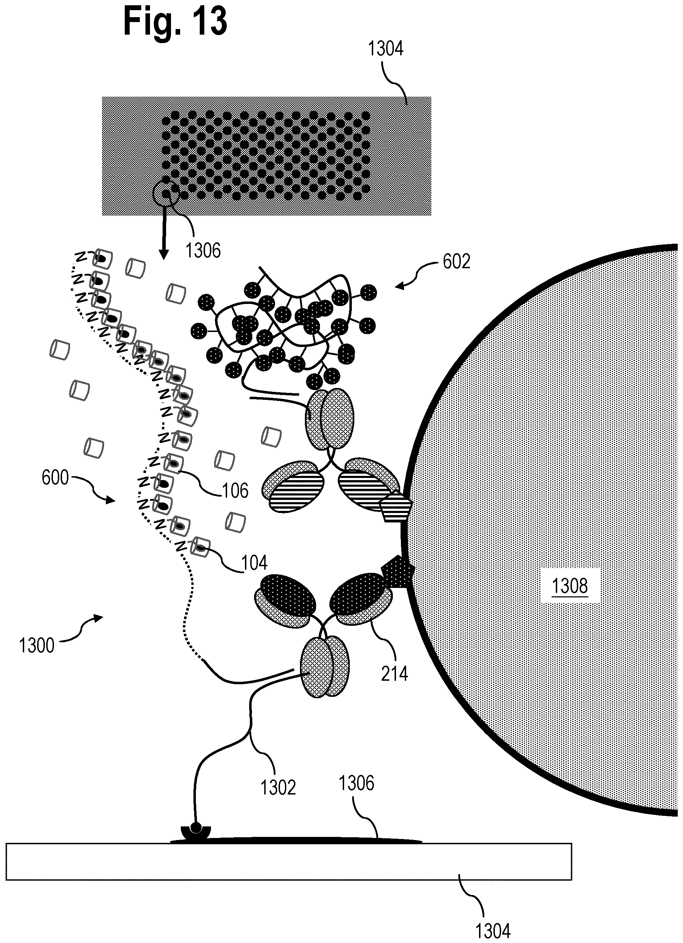

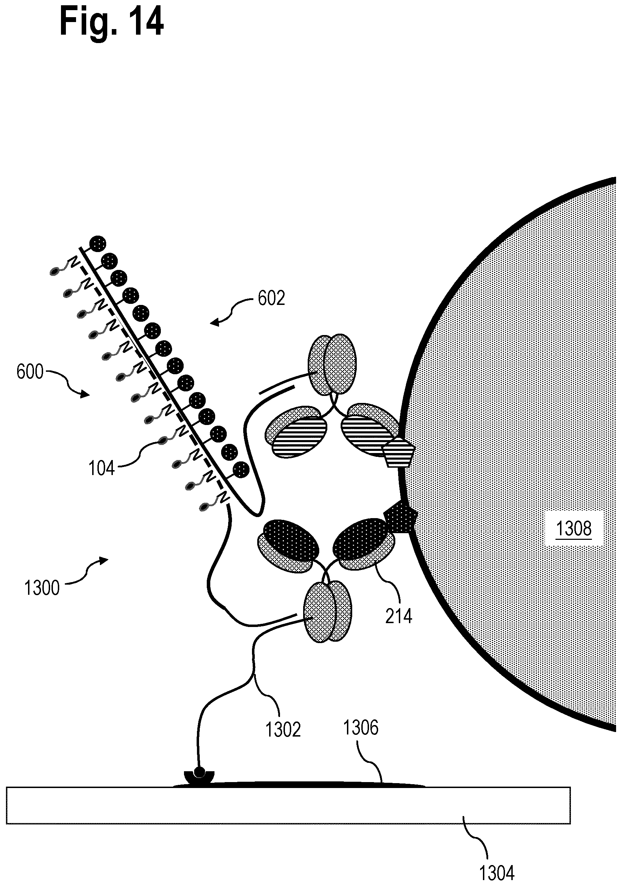

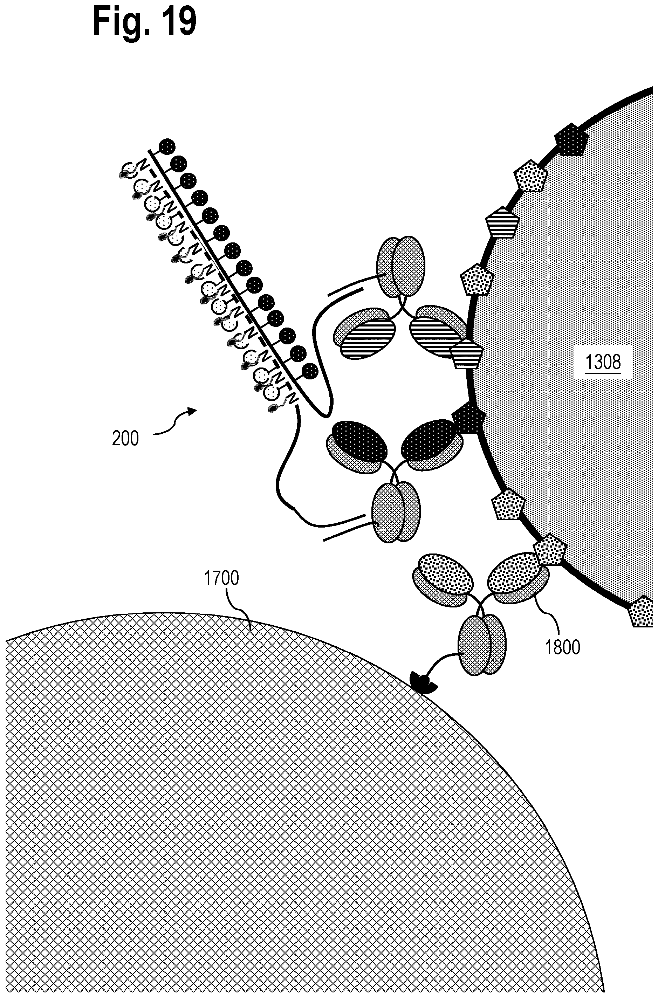

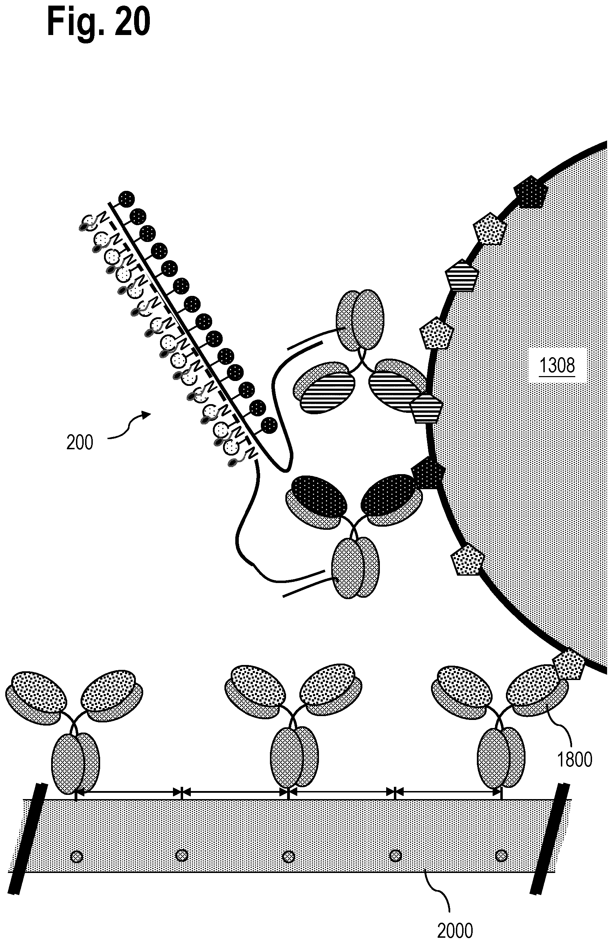

Figures 13 to 22 are schematic views of further embodiments of uses of the disclosed labels and markers. In particular, the Figs. 13 to 17 show markers 1300, 1500, which are attached to a solid support 1304, 1700. To that end, the markers 1300, 1500 comprise an anchor oligonucleotide 1302, which on one end may be (covalently) attached to the antibody 214 of one of the marker parts of the markers 1300, 1500. The other end may be attached to the respective solid support 1304, 1700, for example, a flow cell bottom or a glass slide 1304 e.g. with a plurality of microarrayed spots 1306. An individual spot 1306 may comprise a multiplicity of markers 1300. The marker 1300 may adhere to the spot 1306, e.g. by simply drying down marker part on poly-L-lysine coated glass slides, or may be connected to the spot by way of biotin-streptavidin or any other high affinity interactor pair that is suited to provide the substantially stable conjugation of the marker part to the glass slide. The marker 1300 may comprise the label parts 600, 602 and be configured for dequenching-based hgPHA as shown in the Figures 13 and 14 and/or may be configured for FRET-based hgPHA (not shown). As the first marker part is immobilized this format may be used to capture target analytes 1308, on the first affinity reagent 214, e.g. the first antibody captures an analyte which may be surface protein expressed on a bacterial pathogen (like a pathogenic E.coli strain), or a virus such as the S-protein, or may be a component of exosomes. This embodiment is therefore particularly suited for diagnostic purposes. The hgPHA microarray shown in Figs. 13 and 14 may be brought in contact with the biological sample or multiple biological samples in different ways depending on the intent of the user. In one embodiment the user may wish to measure multiple proteins for example with very high specificity from one biological sample, in this case each spot may comprise a different marker. In other cases, it may be desirable to test a large number of biological samples for the same target analyte 1308. For example, when many test subjects shall be tested for a viral infection such as avian flu, COVID-19, or certain bacterial pathogens, e.g. EHEC, which is then applied to the whole chip either by immersing the hgPHA microarray 1304 or by attaching a flow cell and generating a leak-tight (microfluidic) assembly. The latter is particularly useful, as it allows easy automation and requires on small volumes of samples and reagents. Alternatively, one microarray may comprise spots with different first and/or second marker parts.

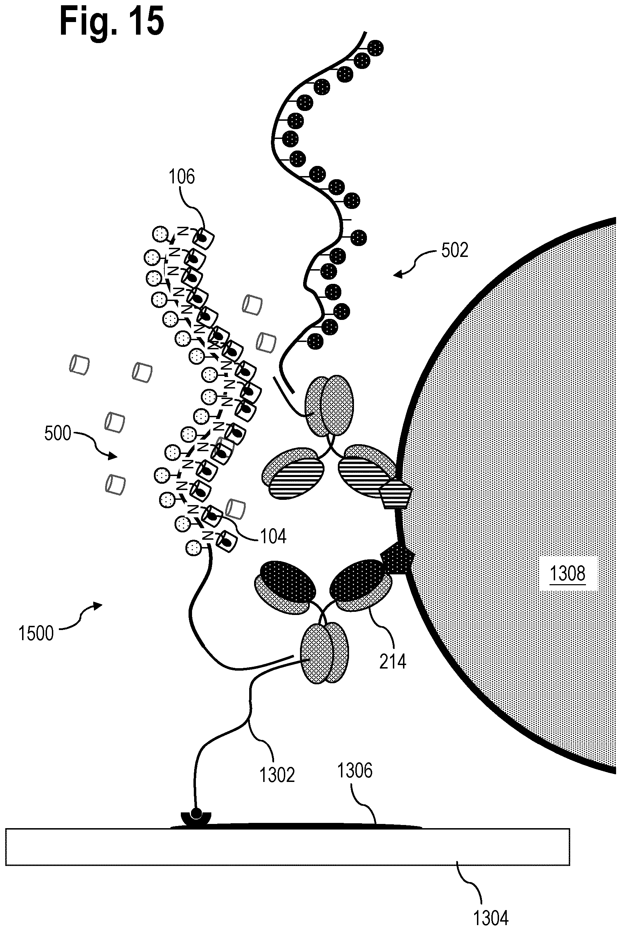

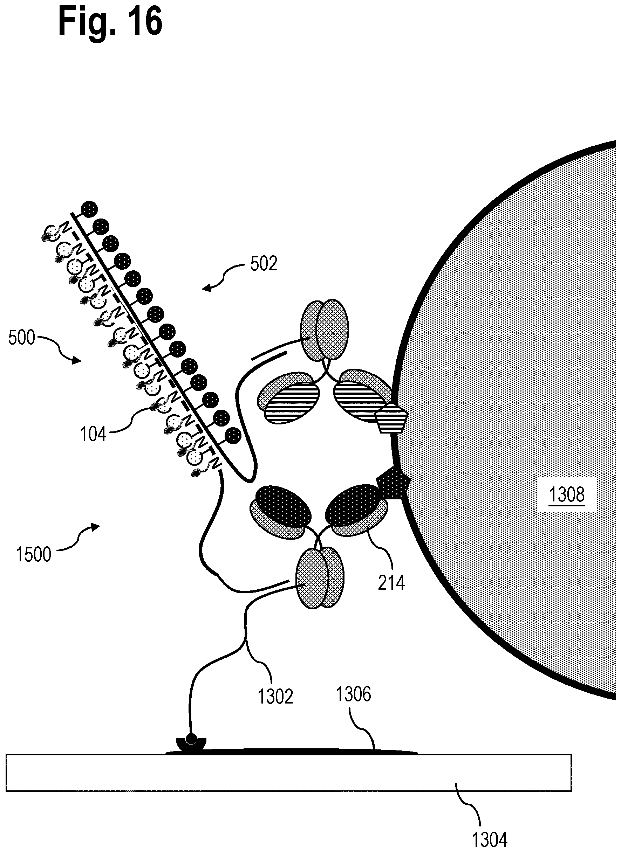

Figure 13 shows the marker 1300 under a complexing condition, where host molecules 106 form a complex with the guest molecules 104 of the label part 600. Figure 14 shows the marker 1300 under a decomplexing condition, when the host molecules 106 have been removed from the marker 1300. Similarly, Figures 15 and 16 show the marker 1500 comprising label parts 500, 502 under a complexing and decomplexing condition, respectively.

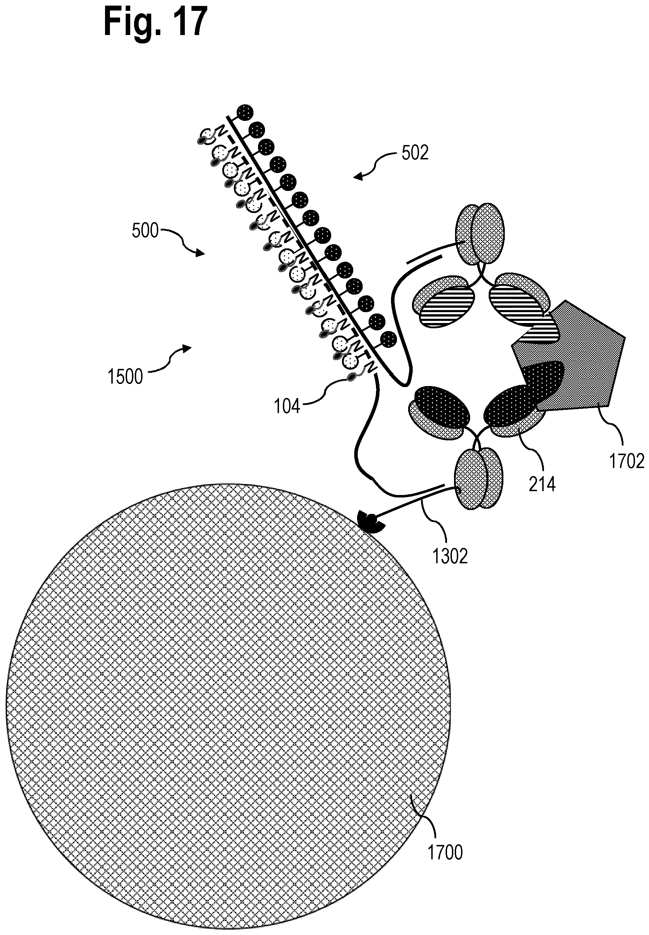

Figure 17 shows the marker 1500 attached to a microbead solid support 1700. The microbead 1700 enables easy processing of the marker 1500, in particular with an attached target analyte 1702, for example, during flow cytometry, sample sorting, or as part of a stationary phase for liquid chromatography.

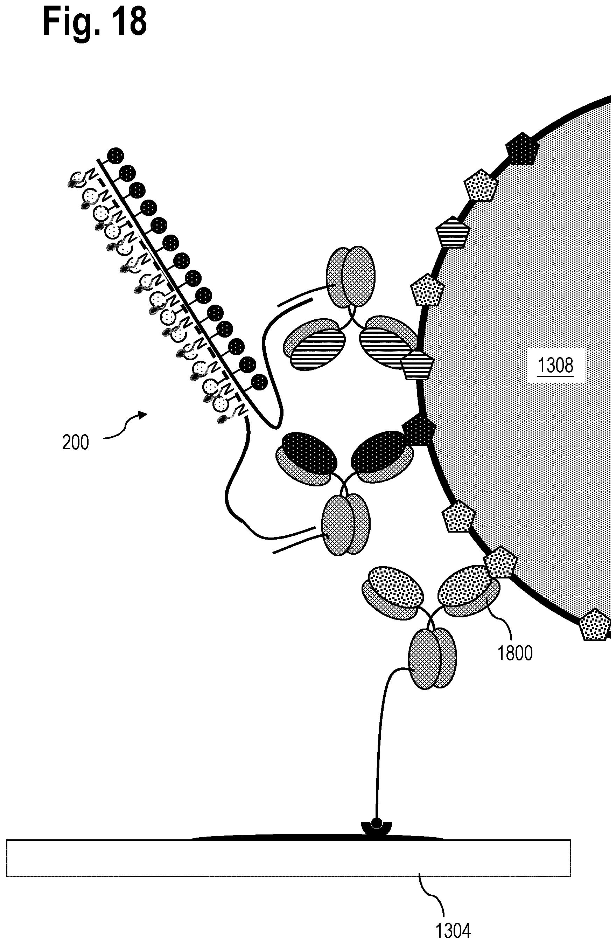

In the embodiments of Figures 18, 19, and 20 the marker 200 is used to bind the target analyte 1308. A tertiary affinity reagent 1800, such as an antibody, which is attached to the solid support 1304, 1700, 2000 is used to capture the target analyte 1308. The solid support 2000 is a nucleic acid based support, in particular a DNA origami based support, e.g. as described in WO 2022/207832 A1 and/or in the European patent application with the application number 24216812.8, the complete contents thereof are included herein by reference. For example, the tertiary affinity reagent 1800 may initially be attached to the solid support 1304, 1700, 2000 in order to capture or bind the target analyte 1308. Subsequently, the presence of the target analyte 1308 may be determined by addition of the marker 200 and generating an optical readout of the solid support 1304, 1700, 2000 with target analyte 1308 and the marker 200.

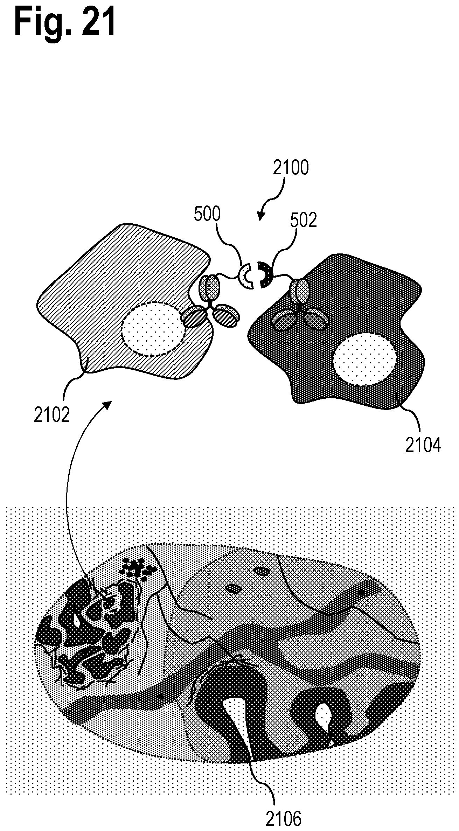

In the embodiment of Figure 21, a marker 2100 comprising the label parts 500, 502, for example, is used to determine the proximity of two cellular target analytes 2102, 2104 of a cellular sample 2106. Marker parts of the marker 2100 may be specific to either one of the cellular target analytes 2102, 2104. Thus, when an optical signal of the marker 2100 can be determined, the target analytes 2102, 2104 are determined to be in close proximity within the sample 2106.

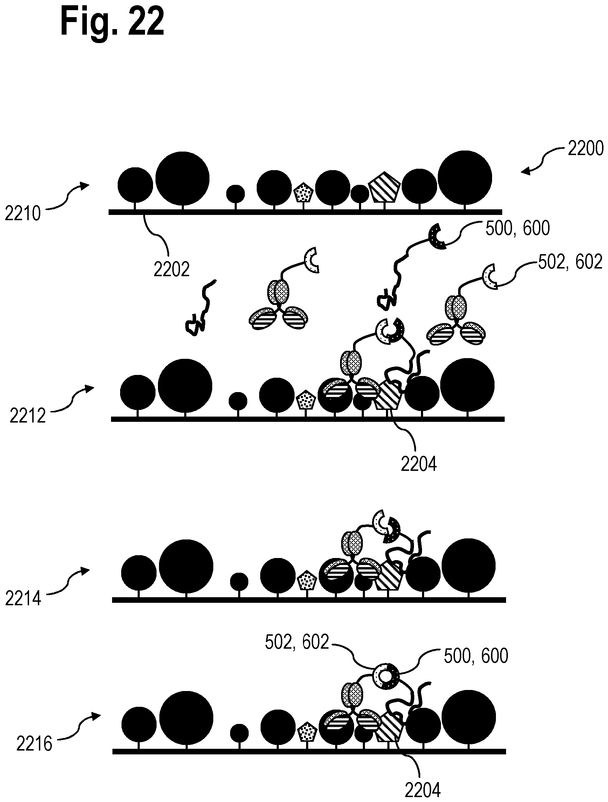

In the embodiment according to Figure 22, analytes 2200 of a sample are immobilised on a solid support 2202 in an initial step 2210. In a next step 2212, first and second marker parts comprising first and second label parts 500, 600, 502, 602, respectively, are contacted with the immobilised analytes 2200 in order to bind the marker parts to respective target analytes 2204. In particular, step 2212 is carried out under a complexing condition. In a next step 2214, any unbound marker parts may be washed away from the solid support 2202. In a next step 2216 a decomplexing condition is applied in order to remove host molecules 106 from the label parts 500, 600. This enables hybridisation between the label parts 500, 600, 502, 602 and detection of the presence of the analytes 2204 in an optical readout. The method described for Figure 22 may be particularly applicable if the target analytes 2204 are of low abundance in the analysed sample.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 206, 504: First nucleic acid backbone
- 102, 210, 506: Second nucleic acid backbone
- 104: Guest molecule
- 106: Host molecule
- 108: Guest-host complex
- 200, 700, 800, 900a, 900b, 900c, 900d, 902a, 902b, 902c, 902d, 1000, 1002, 1204, 1300, 1500, 2100: Marker
- 202: First marker part
- 204: Second marker part
- 208, 508, 604: First labelling moiety
- 212, 510: Second labelling moiety
- 214,: First affinity reagent
- 218: Second affinity reagent
- 216, 2102: First target analyte
- 220, 2104: Second target analyte
- 222, 224: Barcode oligonucleotide
- 500, 600: First label part
- 502, 602: Second label part
- 500a, 600a: Label part decomplexed condition
- 500b, 600b: Label part complexed condition
- 1004, 1006: Secondary affinity reagent
- 1100, 1102, 1104, 1106: Step of method for amplifying barcode oligonucleotides
- 1200: L-adapter oligonucleotide
- 1202: Dendrimeric structure
- 1206: First attachment oligonucleotide
- 1208: Second attachment oligonucleotide
- 1302: Anchor oligonucleotide
- 1304, 1700, 2000, 2202: Solid support
- 1306: Spot
- 1308, 1702, 2204: Target analyte
- 1800: Tertiary affinity reagent
- 2106: Cellular sample
- 2200: Analytes
- 2210, 2212, 2214, 2216: Steps of method for analysing a sample with a marker

## Claims

1. A label for analysing a biological sample comprising:
a first label part comprising a first nucleic acid backbone (100, 206), and
a second label part comprising a second nucleic acid backbone (102, 210), and
wherein the first nucleic acid backbone (100, 206) and the second nucleic acid backbone (102, 210) are each configured to hybridise at least partially to the respective other one,
wherein the label further comprises at least one first labelling moiety (208, 212) and at least one second labelling moiety (212), and
wherein the label further comprises at least one guest molecule (104) configured to form a complex (108) with a host molecule (106).

2. The label according to claim 1, wherein the at least one guest molecule (104) is configured to form the complex (108) with the host molecule (106) under a complexing condition.

3. The label according to one of the preceding claims, wherein the complex (108) is configured to invade a duplex of the first nucleic acid backbone (100, 206) and the second nucleic acid backbone (102, 210) and to reduce the melting temperature of the duplex.

4. The label according to one of the preceding claims, wherein the first nucleic acid backbone (100, 206) and the second nucleic acid backbone (102, 210) each comprise at least one of a natural nucleic acid and a nucleic acid analogue.

5. The label according to one of the preceding claims, wherein the at least one first labelling moiety (208) and/or the at least one second labelling moiety (212) is optically detectable.

6. The label according to one of the preceding claims, wherein the at least one first labelling moiety (208) and/or the at least one second labelling moiety (212) preferably comprise identical fluorescent dyes, or
wherein the at least one first labelling moiety (208) comprises at least one first fluorescent dye and the at least one second labelling moiety (212) comprises at least one second fluorescent dye, the at least one first fluorescent dye and the at least one second fluorescent dye having different characteristics.

7. The label according to one of the preceding claims, wherein the at least one first labelling moiety (208) and the at least one second labelling moiety (212) are configured for non-radiative energy transfer between them.

8. The label according to one of the preceding claims, wherein the at least one first labelling moiety (208) and the at least one second labelling moiety (212) are - preferably covalently - attached to either the first nucleic acid backbone (100, 206) or the second nucleic acid backbone (102, 210).

9. The label according to one of the preceding claims 1 to 7, wherein the at least one first labelling moiety (208) is - preferably covalently - attached to the first nucleic acid backbone (100, 206) and the at least one second labelling moiety (212) is - preferably covalently - attached to the second nucleic acid backbone (102, 210).

10. The label according to one of the preceding claims, wherein the first nucleic acid backbone (100, 206) extends along a first direction and a plurality of the first labelling moieties (208) are arranged on the first nucleic acid backbone (100, 206) along the first direction, and/or the second nucleic acid backbone (102, 210) extends along a second direction and a plurality of the second labelling moieties (212) are arranged on the second nucleic acid backbone (102, 210) along the second direction.

11. The label according to one of the preceding claims, wherein each labelling moiety (208, 212) is substantially equally spaced from any adjacent labelling moiety (208, 212).

12. The label according to one of the preceding claims comprising a plurality of the guest molecules (104), wherein the guest molecules (104) are evenly spaced along the first nucleic acid backbone (100, 206) and/or the second nucleic acid backbone (102, 210).

13. The label according to one of the preceding claims, wherein the at least one guest molecule (104) is one of 1-adamantanemethylamine, ferrocenyl methylamine, 1,4-benzenedimethanamine, and 4-tertbutylbenzylamine.

14. The label according to one of the preceding claims, comprising at least one of the host molecules (106).

15. The label according to one of the preceding claims, wherein the host molecule (106) is a cucurbituril.

16. A marker (200, 500, 600) for analysing a biological sample with a plurality of target analytes (216, 504, 220) comprising:
a label according to one of the preceding claims comprising a first label part and a second label part,
a first marker part (202, 502) comprising a first affinity reagent (214, 503) and the first label part, and
a second marker part (204, 602) comprising a second affinity reagent (218, 604) and the second label part, and
wherein the first affinity reagent (214, 503) and the second affinity reagent (218, 604) are each configured to bind specifically to one of the target analytes (216, 504, 220) of the biological sample.

17. The marker according to claim 16, comprising an anchor oligonucleotide (1302) configured to anchor the marker (1500) to a solid support (1304, 1700, 2000).

18. A method for analysing a biological sample, the method comprising the following steps:
introducing at least one marker (200, 500, 600) according to claims 16 or 17 into the biological sample,
applying a decomplexing condition to the biological sample, and
generating an optical readout of the biological sample with the marker (200, 500, 600).

19. A kit for analysing a biological sample comprising a marker (200, 500, 600) according to claims 16 or 17 and at least one host molecule (106) configured to form a complex with the at least one guest molecule (104) of the marker (200, 500, 600).

20. A use of a label according to one of the claims 1 to 15 or a use of a marker (200, 500, 600) according to claims 16 or 17 or a use of a kit according to claim 18 for a proximity assay for analysing a biological sample.
